# EUROPEAN PATENT APPLICATION

(11) **EP 2 166 107 A1**
(43) Date of publication of application: **24.03.2010**
(21) Application number: 08164220.9
(22) Date of filing: 12.09.2008
(51) Int. Cl.: C12N 15/867, C12N 7/00, C12N 5/10, A01K 67/027, A61K 49/00

(54) **Lentiviral vectors for the expression of shRNA**

(71) Applicant: TaconicArtemis GmbH, 51063 Köln (DE)
(72) Inventor: Brandt, Jens van den, Dr., 37077 Göttingen (DE); Reichardt, Holger, Prof. Dr., 37085 Göttingen (DE); Herold, Marco, Dr., 97082 Würzburg (DE)
(74) Representative: Helbing, Jörg

(57) **Abstract**

The invention provides particular lentiviral vectors that are useful for the transfection of cells with shRNAs. Moreover, cells transfected with such vectors and non-human vertebrates having such cells are provided.

SEQUENCE LISTING

<110>  TaconicArtemis

<120>  Lentiviral Vectors for the Expression of shRNA

<130>  080313ep

<160>  211

<170>  PatentIn version 3.3

<210>  1
<211>  10963
<212>  DNA
<213>  Artificial

<220>
<223>  vector FH1tshRNA-UTG

<400>  1
gaccggctcg ggttctcccg ggacttcgtg gaggacgact tcgccggtgt ggtccgggac      60

gacgtgaccc tgttcatcag cgcggtccag gaccaggtgg tgccggacaa caccctggcc     120

tgggtgtggg tgcgcggcct ggacgagctg tacgccgagt ggtcggaggt cgtgtccacg     180

aacttccggg acgcctccgg gccggccatg accgagatcg gcgagcagcc gtgggggcgg     240

gagttcgccc tgcgcgaccc ggccggcaac tgcgtgcact tcgtggccga ggagcaggac     300

tgacacgtgc tacgagattt cgattccacc gccgccttct atgaaaggtt gggcttcgga     360

atcgttttcc gggacgccgg ctggatgatc ctccagcgcg gggatctcat gctggagttc     420

ttcgcccacc ccaacttgtt tattgcagct tataatggtt acaaataaag caatagcatc     480

acaaatttca caaataaagc atttttttca ctgcattcta gttgtggttt gtccaaactc     540

atcaatgtat cttatcatgt ctgtataccg tcgacctcta gctagagctt ggcgtaatca     600

tggtcatagc tgtttcctgt gtgaaattgt tatccgctca caattccaca caacatacga     660

gccggaagca taaagtgtaa agcctggggt gcctaatgag tgagctaact cacattaatt     720

gcgttgcgct cactgcccgc tttccagtcg ggaaacctgt cgtgccagct gcattaatga     780

atcggccaac gcgcggggag aggcggtttg cgtattgggc gctcttccgc ttcctcgctc     840

actgactcgc tgcgctcggt cgttcggctg cggcgagcgg tatcagctca ctcaaaggcg     900

gtaatacggt tatccacaga atcaggggat aacgcaggaa agaacatgtg agcaaaaggc     960

cagcaaaagg ccaggaaccg taaaaaggcc gcgttgctgg cgtttttcca taggctccgc    1020

ccccctgacg agcatcacaa aaatcgacgc tcaagtcaga ggtggcgaaa cccgacagga    1080

ctataaagat accaggcgtt tccccctgga agctccctcg tgcgctctcc tgttccgacc    1140

ctgccgctta ccggatacct gtccgccttt ctcccttcgg gaagcgtggc gctttctcat    1200

agctcacgct gtaggtatct cagttcggtg taggtcgttc gctccaagct gggctgtgtg    1260

cacgaacccc ccgttcagcc cgaccgctgc gccttatccg gtaactatcg tcttgagtcc 1320

aacccggtaa gacacgactt atcgccactg gcagcagcca ctggtaacag gattagcaga 1380

gcgaggtatg taggcggtgc tacagagttc ttgaagtggt ggcctaacta cggctacact 1440

agaagaacag tatttggtat ctgcgctctg ctgaagccag ttaccttcgg aaaaagagtt 1500

ggtagctctt gatccggcaa acaaaccacc gctggtagcg gtggtttttt tgtttgcaag 1560

cagcagatta cgcgcagaaa aaaaggatct caagaagatc ctttgatctt ttctacgggg 1620

tctgacgctc agtggaacga aaactcacgt taagggattt tggtcatgag attatcaaaa 1680

aggatcttca cctagatcct tttaaattaa aaatgaagtt ttaaatcaat ctaaagtata 1740

tatgagtaaa cttggtctga cagttaccaa tgcttaatca gtgaggcacc tatctcagcg 1800

atctgtctat ttcgttcatc catagttgcc tgactccccg tcgtgtagat aactacgata 1860

cgggagggct taccatctgg ccccagtgct gcaatgatac cgcgagaccc acgctcaccg 1920

gctccagatt tatcagcaat aaaccagcca gccggaaggg ccgagcgcag aagtggtcct 1980

gcaactttat ccgcctccat ccagtctatt aattgttgcc gggaagctag agtaagtagt 2040

tcgccagtta atagtttgcg caacgttgtt gccattgcta caggcatcgt ggtgtcacgc 2100

tcgtcgtttg gtatggcttc attcagctcc ggttcccaac gatcaaggcg agttacatga 2160

tcccccatgt tgtgcaaaaa agcggttagc tccttcggtc ctccgatcgt tgtcagaagt 2220

aagttggccg cagtgttatc actcatggtt atggcagcac tgcataattc tcttactgtc 2280

atgccatccg taagatgctt ttctgtgact ggtgagtact caaccaagtc attctgagaa 2340

tagtgtatgc ggcgaccgag ttgctcttgc ccggcgtcaa tacgggataa taccgcgcca 2400

catagcagaa ctttaaaagt gctcatcatt ggaaaacgtt cttcggggcg aaaactctca 2460

aggatcttac cgctgttgag atccagttcg atgtaaccca ctcgtgcacc caactgatct 2520

tcagcatctt ttactttcac cagcgtttct gggtgagcaa aaacaggaag gcaaaatgcc 2580

gcaaaaaagg gaataagggc gacacggaaa tgttgaatac tcatactctt cctttttcaa 2640

tattattgaa gcatttatca gggttattgt ctcatgagcg gatacatatt tgaatgtatt 2700

tagaaaaata aacaaatagg ggttccgcgc acatttcccc gaaaagtgcc acctgacgtc 2760

gacggatcgg gagatctccc gatcccctat ggtgcactct cagtacaatc tgctctgatg 2820

ccgcatagtt aagccagtat ctgctccctg cttgtgtgtt ggaggtcgct gagtagtgcg 2880

cgagcaaaat ttaagctaca acaaggcaag gcttgaccga caattgcatg aagaatctgc 2940

ttagggttag gcgttttgcg ctgcttcgcg atgtacgggc cagatatacg cgttgacatt 3000

gattattgac tagttattaa tagtaatcaa ttacggggtc attagttcat agcccatata 3060

tggagttccg cgttacataa cttacggtaa atggcccgcc tggctgaccg cccaacgacc 3120

cccgcccatt gacgtcaata atgacgtatg ttcccatagt aacgccaata gggactttcc 3180

attgacgtca atgggtggag tatttacggt aaactgccca cttggcagta catcaagtgt 3240

atcatatgcc aagtacgccc cctattgacg tcaatgacgg taaatggccc gcctggcatt 3300

atgcccagta catgacctta tgggactttc ctacttggca gtacatctac gtattagtca 3360

tcgctattac catggtgatg cggttttggc agtacatcaa tgggcgtgga tagcggtttg 3420

actcacgggg atttccaagt ctccacccca ttgacgtcaa tgggagtttg ttttggcacc 3480

aaaatcaacg ggactttcca aaatgtcgta acaactccgc cccattgacg caaatgggcg 3540

gtaggcgtgt acggtgggag gtctatataa gcagcgcgtt ttgcctgtac tgggtctctc 3600

tggttagacc agatctgagc ctgggagctc tctggctaac tagggaaccc actgcttaag 3660

cctcaataaa gcttgccttg agtgcttcaa gtagtgtgtg cccgtctgtt gtgtgactct 3720

ggtaactaga gatccctcag acccttttag tcagtgtgga aaatctctag cagtggcgcc 3780

cgaacaggga cttgaaagcg aaagggaaac cagaggagct ctctcgacgc aggactcggc 3840

ttgctgaagc gcgcacggca agaggcgagg ggcggcgact ggtgagtacg ccaaaaattt 3900

tgactagcgg aggctagaag gagagagatg ggtgcgagag cgtcagtatt aagcggggga 3960

gaattagatc gcgatgggaa aaaattcggt taaggccagg gggaaagaaa aaatataaat 4020

taaaacatat agtatgggca agcagggagc tagaacgatt cgcagttaat cctggcctgt 4080

tagaaacatc agaaggctgt agacaaatac tgggacagct acaaccatcc cttcagacag 4140

gatcagaaga acttagatca ttatataata cagtagcaac cctctattgt gtgcatcaaa 4200

ggatagagat aaaagacacc aaggaagctt tagacaagat agaggaagag caaaacaaaa 4260

gtaagaccac cgcacagcaa gcggccgctg atcttcagac ctggaggagg agatatgagg 4320

gacaattgga gaagtgaatt atataaatat aaagtagtaa aaattgaacc attaggagta 4380

gcacccacca aggcaaagag aagagtggtg cagagagaaa aaagagcagt gggaatagga 4440

gctttgttcc ttgggttctt gggagcagca ggaagcacta tgggcgcagc gtcaatgacg 4500

ctgacggtac aggccagaca attattgtct ggtatagtgc agcagcagaa caatttgctg 4560

agggctattg aggcgcaaca gcatctgttg caactcacag tctggggcat caagcagctc 4620

caggcaagaa tcctggctgt ggaaagatac ctaaaggatc aacagctcct ggggatttgg 4680

ggttgctctg gaaaactcat ttgcaccact gctgtgcctt ggaatgctag ttggagtaat 4740

aaatctctgg aacagatttg gaatcacacg acctggatgg agtgggacag agaaattaac 4800

aattacacaa gcttaataca ctccttaatt gaagaatcgc aaaaccagca agaaaagaat 4860

gaacaagaat tattggaatt agataaatgg gcaagtttgt ggaattggtt taacataaca 4920

aattggctgt ggtatataaa attattcata atgatagtag gaggcttggt aggtttaaga 4980

atagtttttg ctgtactttc tatagtgaat agagttaggc agggatattc accattatcg 5040

tttcagaccc acctcccaac cccgagggga cccgacaggc ccgaaggaat agaagaagaa 5100

ggtggagaga gagacagaga cagatccatt cgattagtga acggatcggc actgcgtgcg 5160

ccaattctgc agacaaatgg cagtattcat ccacaatttt aaaagaaaag gggggattgg 5220

ggggtacagt gcaggggaaa gaatagtaga cataatagca acagacatac aaactaaaga 5280

attacaaaaa caaattacaa aaattcaaaa ttttcgggtt tattacaggg acagcagaga 5340

tccagtttgg ttaattaacc atggaattcg aacgctgacg tcatcaaccc gctccaagga 5400

atcgcgggcc cagtgtcact aggcgggaac acccagcgcg cgtgcgccct ggcaggaaga 5460

tggctgtgag ggacagggga gtggcgccct gcaatatttg catgtcgcta tgtgttctgg 5520

gaaatcacca taaacgtgaa atgtctttgg atttgggaat cttataagtc cctatcagtg 5580

atagagattc ccagaccaga cccgaagatt tcttcaagag agaaatcttc gggtctggtc 5640

ttttttggcg cggctagtta ttaatagtaa tcaattacgg ggtcattagt tcatagccca 5700

tattaattaa gggtgcagcg gcctccgcgc cgggttttgg cgcctcccgc gggcgccccc 5760

ctcctcacgg cgagcgctgc cacgtcagac gaagggcgca ggagcgttcc tgatccttcc 5820

gcccggacgc tcaggacagc ggcccgctgc tcataagact cggccttaga accccagtat 5880

cagcagaagg acattttagg acgggacttg ggtgactcta gggcactggt tttctttcca 5940

gagagcggaa caggcgagga aaagtagtcc cttctcggcg attctgcgga gggatctccg 6000

tggggcggtg aacgccgatg attatataag gacgcgccgg gtgtggcaca gctagttccg 6060

tcgcagccgg gatttgggtc gcggttcttg tttgtggatc gctgtgatcg tcacttggtg 6120

agttgcgggc tgctgggctg gccggggctt tcgtggccgc cgggccgctc ggtgggacgg 6180

aagcgtgtgg agagaccgcc aagggctgta gtctgggtcc gcgagcaagg ttgccctgaa 6240

ctgggggttg gggggagcgc acaaaatggc ggctgttccc gagtcttgaa tggaagacgc 6300

ttgtaaggcg ggctgtgagg tcgttgaaac aaggtggggg gcatggtggg cggcaagaac 6360

ccaaggtctt gaggccttcg ctaatgcggg aaagctctta ttcgggtgag atgggctggg 6420

gcaccatctg gggaccctga cgtgaagttt gtcactgact ggagaactcg ggtttgtcgt 6480

ctggttgcgg gggcggcagt tatgcggtgc cgttgggcag tgcacccgta cctttgggag 6540

cgcgcgcctc gtcgtgtcgt gacgtcaccc gttctgttgg cttataatgc agggtggggc 6600

cacctgccgg taggtgtgcg gtaggctttt ctccgtcgca ggacgcaggg ttcgggccta 6660

gggtaggctc tcctgaatcg acaggcgccg gacctctggt gaggggaggg ataagtgagg 6720

cgtcagtttc tttggtcggt tttatgtacc tatcttctta agtagctgaa gctccggttt 6780

tgaactatgc gctcggggtt ggcgagtgtg ttttgtgaag ttttttaggc accttttgaa 6840

atgtaatcat ttgggtcaat atgtaatttt cagtgttaga ctagtaaagc ttctgcaggt 6900

cgactctaga aaattgtccg ctaaattctg gccgtttttg gcttttttgt tagacaggat 6960

ccatgtccag actggacaag agcaaagtca tcaactctgc cttggagctc ctgaatgaag 7020

ttggcattga gggcttgacc accaggaagc tggcccagaa gctgggtgtg gagcagccta 7080

ccctgtactg gcatgtgaag aacaagaggg ctctgcttga tgccctggcc attgagatgt 7140

tggacaggca ccacacccac ttctgccctc tggaagggga gtcctggcag gacttcctga 7200

ggaacaatgc caagagcttc agatgtgcct tgctctccca ccgggatggt gccaaagttc 7260

acttgggcac caggcctaca gagaagcagt atgagaccct ggagaaccag ctggcattcc 7320

tgtgccaaca aggcttctcc ctggaaaatg ccttgtatgc cctctctgct gtgggccact 7380

tcaccttggg ctgtgtgctg gaggaccagg agcaccaagt tgccaaggag gagagggaga 7440

cccccaccac tgactccatg ccaccactgc tgcggcaagc tattgagttg tttgaccacc 7500

aaggggctga gcctgcattc ctttttggcc tggaactgat catctgtggc ctggaaaagc 7560

agctgaaatg tgagtctggc tctgggtccg gtgagggcag aggaagtctg ctaacatgcg 7620

gtgacgtcga ggagaatcct ggcccaatgg tgagcaaggg cgaggagctg ttcaccgggg 7680

tggtgcccat cctggtcgag ctggacggcg acgtaaacgg ccacaagttc agcgtgtccg 7740

gcgagggcga gggcgatgcc acctacggca agctgaccct gaagttcatc tgcaccaccg 7800

gcaagctgcc cgtgccctgg cccaccctcg tgaccaccct gacctacggc gtgcagtgct 7860

tcagccgcta ccccgaccac atgaagcagc acgacttctt caagtccgcc atgcccgaag 7920

gctacgtcca ggagcgcacc atcttcttca aggacgacgg caactacaag acccgcgccg 7980

aggtgaagtt cgagggcgac accctggtga accgcatcga gctgaagggc atcgacttca 8040

aggaggacgg caacatcctg gggcacaagc tggagtacaa ctacaacagc cacaacgtct 8100

atatcatggc cgacaagcag aagaacggca tcaaggtgaa cttcaagatc cgccacaaca 8160

tcgaggacgg cagcgtgcag ctcgccgacc actaccagca gaacaccccc atcggcgacg 8220

gccccgtgct gctgcccgac aaccactacc tgagcaccca gtccgccctg agcaaagacc 8280

ccaacgagaa gcgcgatcac atggtcctgc tggagttcgt gaccgccgcc gggatcactc 8340

tcggcatgga cgagctgtac aagtaaagcg gccgcgactc tacaattcga tatcaagctt 8400

atcgataatc aacctctgga ttacaaaatt tgtgaaagat tgactggtat tcttaactat 8460

gttgctcctt ttacgctatg tggatacgct gctttaatgc ctttgtatca tgctattgct 8520

tcccgtatgg ctttcatttt ctcctccttg tataaatcct ggttgctgtc tctttatgag 8580

gagttgtggc ccgttgtcag gcaacgtggc gtggtgtgca ctgtgtttgc tgacgcaacc 8640

cccactggtt ggggcattgc caccacctgt cagctccttt ccgggacttt cgctttcccc 8700

ctccctattg ccacggcgga actcatcgcc gcctgccttg cccgctgctg gacaggggct 8760

cggctgttgg gcactgacaa ttccgtggtg ttgtcgggga aatcatcgtc ctttccttgg 8820

ctgctcgcct gtgttgccac ctggattctg cgcgggacgt ccttctgcta cgtcccttcg 8880

gccctcaatc cagcggacct tccttcccgc ggcctgctgc cggctctgcg gcctcttccg 8940

cgtcttcgcc ttcgccctca gacgagtcgg atctcccttt gggccgcctc cccgcatcga 9000

taccgtcgac ctcgatcgag gacctagaaa aacatggagc aatcacaagt agcaatacag 9060

cagctaccaa tgctgattgt gcctggctag aagcacaaga ggaggaggag gtgggttttc 9120

cagtcacacc tcaggtacct ttaagaccaa tgacttacaa ggcagctgta gatcttagcc 9180

actttttaaa agaaaagggg ggactggaag ggctaattca ctcccaacga agacaagata 9240

tccttgatct gtggatctac cacacacaag gctacttccc tgattggcag aactacacac 9300

cagggccagg gatcagatat ccactgacct ttggatggtg ctacaagcta gtaccagttg 9360

agcaagagaa ggtagaagaa gccaatgaag gagagaacac ccgcttgtta caccctgtga 9420

gcctgcatgg gatggatgac ccggagagag aagtattaga gtggaggttt gacagccgcc 9480

tagcatttca tcacatggcc cgagagctgc atccggactg tactgggtct ctctggttag 9540

accagatctg agcctgggag ctctctggct aactagggaa cccactgctt aagcctcaat 9600

aaagcttgcc ttgagtgctt caagtagtgt gtgcccgtct gttgtgtgac tctggtaact 9660

agagatccct cagacccttt tagtcagtgt ggaaaatctc tagcagggcc cgtttaaacc 9720

cgctgatcag cctcgactgt gccttctagt tgccagccat ctgttgtttg cccctccccc 9780

gtgccttcct tgaccctgga aggtgccact cccactgtcc tttcctaata aaatgaggaa 9840

attgcatcgc attgtctgag taggtgtcat tctattctgg ggggtggggt ggggcaggac 9900

agcaaggggg aggattggga agacaatagc aggcatgctg gggatgcggt gggctctatg 9960

gcttctgagg cggaaagaac cagctggggc tctagggggt atccccacgc gccctgtagc 10020

ggcgcattaa gcgcggcggg tgtggtggtt acgcgcagcg tgaccgctac acttgccagc 10080

gccctagcgc ccgctccttt cgctttcttc ccttcctttc tcgccacgtt cgccggcttt 10140

ccccgtcaag ctctaaatcg ggggctccct ttagggttcc gatttagtgc tttacggcac 10200

ctcgacccca aaaaacttga ttagggtgat ggttcacgta gtgggccatc gccctgatag 10260

acggtttttc gccctttgac gttggagtcc acgttcttta atagtggact cttgttccaa 10320

actggaacaa cactcaaccc tatctcggtc tattcttttg atttataagg gattttgccg 10380

atttcggcct attggttaaa aaatgagctg atttaacaaa aatttaacgc gaattaattc 10440

tgtggaatgt gtgtcagtta gggtgtggaa agtccccagg ctccccagca ggcagaagta 10500

tgcaaagcat gcatctcaat tagtcagcaa ccaggtgtgg aaagtcccca ggctccccag 10560

caggcagaag tatgcaaagc atgcatctca attagtcagc aaccatagtc ccgcccctaa 10620

ctccgcccat cccgccccta actccgccca gttccgccca ttctccgccc catggctgac 10680

taattttttt tatttatgca gaggccgagg ccgcctctgc ctctgagcta ttccagaagt 10740

agtgaggagg cttttttgga ggcctaggct tttgcaaaaa gctcccggga gcttgtatat 10800

ccattttcgg atctgatcag cacgtgttga caattaatca tcggcatagt atatcggcat 10860

agtataatac gacaaggtga ggaactaaac catggccaag ttgaccagtg ccgttccggt 10920

gctcaccgcg cgcgacgtcg ccggagcggt cgagttctgg acc 10963

<210> 2
<211> 53
<212> DNA
<213> Artificial

<220>
<223> shCD8 alpha sequence

<400> 2
cggagcaagc tgaacgatat atttcaagag agtatatcgt tcagcttgct ccg 53

<210> 3
<211> 20
<212> DNA
<213> Artificial

<220>
<223> probe

<400> 3
gaccagaccc gaagatttct 20

<210> 4
<211> 32
<212> DNA
<213> Artificial

<220>
<223> probe

<400> 4
tcctgcaatt cacattaatt ctcgcagcta gc 32

<210> 5
<211> 66
<212> DNA
<213> Foot-and-mouth disease virus

<400> 5
gtgaaacaga ctttgaattt tgaccttctc aagttggcgg gagacgtgga gtccaaccca 60

gggccc 66

<210> 6
<211> 60
<212> DNA
<213> equine rhinitis A virus

<400> 6

caatgtacta actacgcttt gttgaaactc gctggcgatg ttgaaagtaa ccccggtcct     60

<210>  7
<211>  47
<212>  DNA
<213>  Artificial

<220>
<223>  shRNA

<400>  7
tgagaagtct cccagtcagt tcaagagact gactgggaga cttctca                   47

<210>  8
<211>  47
<212>  DNA
<213>  Artificial

<220>
<223>  shRNA

<400>  8
gactccagtg gtaatctact tcaagagagt agattaccac tggagtc                   47

<210>  9
<211>  47
<212>  DNA
<213>  Artificial

<220>
<223>  shRNA

<400>  9
cggcaggact ccgggccgat tcaagagatc ggcccggagt cctgccg                   47

<210>  10
<211>  47
<212>  DNA
<213>  Artificial

<220>
<223>  shRNA

<400>  10
cctcatgcag ttctctttgt tcaagagaca aagagaactg catgagg                   47

<210>  11
<211>  50
<212>  DNA
<213>  Artificial

<220>
<223>  shRNA

<400>  11
aagatgaagc cactccctat ttcaagagaa aatagggagt ggcttcatct                50

<210>  12
<211>  41
<212>  DNA
<213>  Artificial

<220>
<223>  shRNA

<400>  12
gacagagcca agtggactca cagagtccac ttggctctgt c                          41


<210>  13
<211>  42
<212>  DNA
<213>  Artificial

<220>
<223>  shRNA

<400>  13
ctggacttcc agaagaacat tcgtgttctt ctggaagtcc ag                         42


<210>  14
<211>  47
<212>  DNA
<213>  Artificial

<220>
<223>  shRNA

<400>  14
gagattggtc cagaacagtt tcaagagaac tgttctggac caatctc                    47


<210>  15
<211>  47
<212>  DNA
<213>  Artificial

<220>
<223>  shRNA

<400>  15
gcccttccga tcatggtagt tcaagagact accatgatcg gaagggc                    47


<210>  16
<211>  47
<212>  DNA
<213>  Artificial

<220>
<223>  shRNA

<400>  16
tctttagaat tcttaagtat tcaagagata cttaagaatt ctaaaga                    47


<210>  17
<211>  47
<212>  DNA

<213>  Artificial

<220>
<223>  shRNA

<400>  17
cattagctat atcaacatgt tcaagagaca tgttgatata gctaatg                47


<210>  18
<211>  47
<212>  DNA
<213>  Artificial

<220>
<223>  shRNA

<400>  18
accacaaacg gcggaacgat tcaagagatc gttccgccgt ttgtggt                47


<210>  19
<211>  47
<212>  DNA
<213>  Artificial

<220>
<223>  shRNA

<400>  19
gagggtcttg gaggtcttct tcaagagaga agacctccaa gaccctc                47


<210>  20
<211>  47
<212>  DNA
<213>  Artificial

<220>
<223>  shRNA

<400>  20
gtccatgccc agccgtacat tcaagagatg tacggctggg catggac                47


<210>  21
<211>  47
<212>  DNA
<213>  Artificial

<220>
<223>  shRNA

<400>  21
gctggacacc ctcgtggagt tcaagagact ccacgagggt gtccagc                47


<210>  22
<211>  47
<212>  DNA
<213>  Artificial

<220>

<223>  shRNA

<400>  22
gaatatcaga gaattgagtt tcaagagaac tcaattctct gatattc                    47


<210>  23
<211>  47
<212>  DNA
<213>  Artificial

<220>
<223>  shRNA

<400>  23
tggacttcat gaggaaatgt tcaagagaca tttcctcatg aagtcca                    47


<210>  24
<211>  47
<212>  DNA
<213>  Artificial

<220>
<223>  shRNA

<400>  24
tattgaatat cctgtggact tcaagagagt ccacaggata ttcaata                    47


<210>  25
<211>  47
<212>  DNA
<213>  Artificial

<220>
<223>  shRNA

<400>  25
ttgtactgag agaaactgct tcaagagagc agtttctctc agtacaa                    47


<210>  26
<211>  47
<212>  DNA
<213>  Artificial

<220>
<223>  shRNA

<400>  26
gatcaatgat aggtttgaat tcaagagatt caaacctatc attgatc                    47


<210>  27
<211>  47
<212>  DNA
<213>  Artificial

<220>
<223>  shRNA

<400>  27

ggagtttgag aagtttaaat tcaagagatt taaacttctc aaactcc 47

<210> 28
<211> 47
<212> DNA
<213> Artificial

<220>
<223> shRNA

<400> 28
gaactcctcg cttgctgagt tcaagagact cagcaagcga ggagttc 47

<210> 29
<211> 47
<212> DNA
<213> Artificial

<220>
<223> shRNA

<400> 29
ccgaatttaa cagagagaat tcaagagatt ctctctgtta aattcgg 47

<210> 30
<211> 47
<212> DNA
<213> Artificial

<220>
<223> shRNA

<400> 30
gacagcagaa gaatgcagat tcaagagatc tgcattcttc tgctgtc 47

<210> 31
<211> 47
<212> DNA
<213> Artificial

<220>
<223> shRNA

<400> 31
ataaagctca acgagaacct tcaagagagg ttctcgttga gctttat 47

<210> 32
<211> 47
<212> DNA
<213> Artificial

<220>
<223> shRNA

<400> 32
ggtgaagtgg cagaagaatt tcaagagaat tcttctgcca cttcacc 47

<210>  33
<211>  47
<212>  DNA
<213>  Artificial

<220>
<223>  shRNA

<400>  33
gtattgcagt aatcatcact tcaagagagt gatgattact gcaatac                    47


<210>  34
<211>  47
<212>  DNA
<213>  Artificial

<220>
<223>  shRNA

<400>  34
gatatggggt tccatgtcat tcaagagatg acatggaacc ccatatc                    47


<210>  35
<211>  47
<212>  DNA
<213>  Artificial

<220>
<223>  shRNA

<400>  35
ggagacatgg ttcttagtgt tcaagagaca ctaagaacca tgtctcc                    47


<210>  36
<211>  47
<212>  DNA
<213>  Artificial

<220>
<223>  shRNA

<400>  36
agcaccaagt tcgtctcagt tcaagagact gagacgaact tggtgct                    47


<210>  37
<211>  47
<212>  DNA
<213>  Artificial

<220>
<223>  shRNA

<400>  37
gatgcaacac tgaaagaact tcaagagagt tctttcagtg ttgcatc                    47


<210>  38
<211>  47
<212>  DNA

<213>  Artificial

<220>
<223>  shRNA

<400>  38
gtcaatggca gtgatgatat tcaagagata tcatcactgc cattgac                47


<210>  39
<211>  47
<212>  DNA
<213>  Artificial

<220>
<223>  shRNA

<400>  39
cctgctagct gcctgtggct tcaagagagc cacaggcagc tagcagg                47


<210>  40
<211>  47
<212>  DNA
<213>  Artificial

<220>
<223>  shRNA

<400>  40
ccacctttgc cagaaggagt tcaagagact ccttctggca aaggtgg                47


<210>  41
<211>  47
<212>  DNA
<213>  Artificial

<220>
<223>  shRNA

<400>  41
ccctattgag gcaagtgtct tcaagagaga cacttgcctc aataggg                47


<210>  42
<211>  47
<212>  DNA
<213>  Artificial

<220>
<223>  shRNA

<400>  42
gaaggaaaac ttgctgacgt tcaagagacg tcagcaagtt ttccttc                47


<210>  43
<211>  47
<212>  DNA
<213>  Artificial

<220>

<223>  shRNA

<400>  43
ctcacctggg tccatgagat tcaagagatc tcatggaccc aggtgag                    47


<210>  44
<211>  47
<212>  DNA
<213>  Artificial

<220>
<223>  shRNA

<400>  44
gctgtcttac cgtgtggtct tcaagagaga ccacacggta agacagc                    47


<210>  45
<211>  47
<212>  DNA
<213>  Artificial

<220>
<223>  shRNA

<400>  45
cctggaccgc atgtatgact tcaagagagt catacatgcg gtccagg                    47


<210>  46
<211>  47
<212>  DNA
<213>  Artificial

<220>
<223>  shRNA

<400>  46
gtcaatggca gtgatgatat tcaagagata tcatcactgc cattgac                    47


<210>  47
<211>  47
<212>  DNA
<213>  Artificial

<220>
<223>  shRNA

<400>  47
cctgctagct gcctgtggct tcaagagagc cacaggcagc tagcagg                    47


<210>  48
<211>  47
<212>  DNA
<213>  Artificial

<220>
<223>  shRNA

<400>  48

ccacctttgc cagaaggagt tcaagagact ccttctggca aaggtgg                47

<210>  49
<211>  47
<212>  DNA
<213>  Artificial

<220>
<223>  shRNA

<400>  49
ccctattgag gcaagtgtct tcaagagaga cacttgcctc aataggg                47

<210>  50
<211>  47
<212>  DNA
<213>  Artificial

<220>
<223>  shRNA

<400>  50
ggaaatccga attgcttggt tcaagagacc aagcaattcg gatttcc                47

<210>  51
<211>  47
<212>  DNA
<213>  Artificial

<220>
<223>  shRNA

<400>  51
cacatttctt caagtgtggt tcaagagacc acacttgaag aaatgtg                47

<210>  52
<211>  47
<212>  DNA
<213>  Artificial

<220>
<223>  shRNA

<400>  52
cagcaggatg ctcaagaatt tcaagagaat tcttgagcat cctgctg                47

<210>  53
<211>  47
<212>  DNA
<213>  Artificial

<220>
<223>  shRNA

<400>  53
gctgaatacc tacattggct tcaagagagc caatgtaggt attcagc                47

<210>  54
<211>  47
<212>  DNA
<213>  Artificial

<220>
<223>  shRNA

<400>  54
gggcttgtgc ctggccttgt tcaagagaca aggccaggca caagccc                47


<210>  55
<211>  47
<212>  DNA
<213>  Artificial

<220>
<223>  shRNA

<400>  55
gccttgtcct gccaagaagt tcaagagact tcttggcagg acaaggc                47


<210>  56
<211>  47
<212>  DNA
<213>  Artificial

<220>
<223>  shRNA

<400>  56
gattgaagcc aagggaacgt tcaagagacg ttcccttggc ttcaatc                47


<210>  57
<211>  47
<212>  DNA
<213>  Artificial

<220>
<223>  shRNA

<400>  57
tggcgcctgc tccccatctt tcaagagaag atggggagca ggcgcca                47


<210>  58
<211>  47
<212>  DNA
<213>  Artificial

<220>
<223>  shRNA

<400>  58
gaaccagcag gctctgtggt tcaagagacc acagagcctg ctggttc                47


<210>  59
<211>  47
<212>  DNA

<213>  Artificial

<220>
<223>  shRNA

<400>  59
ggaagcataa ttatctgcct tcaagagagg cagataatta tgcttcc                    47


<210>  60
<211>  47
<212>  DNA
<213>  Artificial

<220>
<223>  shRNA

<400>  60
agaagaagat gcttttcact tcaagagagt gaaaagcatc ttcttct                    47


<210>  61
<211>  47
<212>  DNA
<213>  Artificial

<220>
<223>  shRNA

<400>  61
cttgcagagg aggaacccat tcaagagatg ggttcctcct ctgcaag                    47


<210>  62
<211>  47
<212>  DNA
<213>  Artificial

<220>
<223>  shRNA

<400>  62
gcaaacaatc agcaatgcct tcaagagagg cattgctgat tgtttgc                    47


<210>  63
<211>  47
<212>  DNA
<213>  Artificial

<220>
<223>  shRNA

<400>  63
ttggactgat tcatgctatt tcaagagaat agcatgaatc agtccaa                    47


<210>  64
<211>  47
<212>  DNA
<213>  Artificial

<220>

<223>  shRNA

<400>  64
ctggcaattc gttgatgtat tcaagagata catcaacgaa ttgccag                47


<210>  65
<211>  47
<212>  DNA
<213>  Artificial

<220>
<223>  shRNA

<400>  65
ttagatgggc ggaagccatt tcaagagaat ggcttccgcc catctaa                47


<210>  66
<211>  47
<212>  DNA
<213>  Artificial

<220>
<223>  shRNA

<400>  66
gaggagtctc tgggctcggt tcaagagacc gagcccagag actcctc                47


<210>  67
<211>  47
<212>  DNA
<213>  Artificial

<220>
<223>  shRNA

<400>  67
gagctgaagg gacaagaagt tcaagagact tcttgtccct tcagctc                47


<210>  68
<211>  47
<212>  DNA
<213>  Artificial

<220>
<223>  shRNA

<400>  68
tgtcgggtag atgacaaggt tcaagagacc ttgtcatcta cccgaca                47


<210>  69
<211>  47
<212>  DNA
<213>  Artificial

<220>
<223>  shRNA

<400>  69

cacagctgtt cttctgttct tcaagagaga acagaagaac agctgtg 47

<210> 70
<211> 47
<212> DNA
<213> Artificial

<220>
<223> shRNA

<400> 70
gtggaagcct ttacagatct tcaagagaga tctgtaaagg cttccac 47

<210> 71
<211> 47
<212> DNA
<213> Artificial

<220>
<223> shRNA

<400> 71
caacagctgc cttcatctgt tcaagagaca gatgaaggca gctgttg 47

<210> 72
<211> 47
<212> DNA
<213> Artificial

<220>
<223> shRNA

<400> 72
ccataggcag tcctcctaat tcaagagatt aggaggactg cctatgg 47

<210> 73
<211> 47
<212> DNA
<213> Artificial

<220>
<223> shRNA

<400> 73
tgtatcactg ccactggttt tcaagagaaa ccagtggcag tgataca 47

<210> 74
<211> 47
<212> DNA
<213> Artificial

<220>
<223> shRNA

<400> 74
catgttgggc agctgcagct tcaagagagc tgcagctgcc caacatg 47

<210>  75
<211>  47
<212>  DNA
<213>  Artificial

<220>
<223>  shRNA

<400>  75
cacaactgga gacctgaagt tcaagagact tcaggtctcc agttgtg                47


<210>  76
<211>  47
<212>  DNA
<213>  Artificial

<220>
<223>  shRNA

<400>  76
gtatgcctcc aagaaagagt tcaagagact ctttcttgga ggcatac                47


<210>  77
<211>  47
<212>  DNA
<213>  Artificial

<220>
<223>  shRNA

<400>  77
cttcacagta catttctctt tcaagagaag agaaatgtac tgtgaag                47


<210>  78
<211>  47
<212>  DNA
<213>  Artificial

<220>
<223>  shRNA

<400>  78
gtactttcaa ggccggggtt tcaagagaac cccggccttg aaagtac                47


<210>  79
<211>  47
<212>  DNA
<213>  Artificial

<220>
<223>  shRNA

<400>  79
cttggacaag caagccaaat tcaagagatt tggcttgctt gtccaag                47


<210>  80
<211>  47
<212>  DNA

<213>  Artificial

<220>
<223>  shRNA

<400>  80
gactattgtg actgatgttt tcaagagaaa catcagtcac aatagtc                47


<210>  81
<211>  47
<212>  DNA
<213>  Artificial

<220>
<223>  shRNA

<400>  81
ggagaacttt ctgaagcgct tcaagagagc gcttcagaaa gttctcc                47


<210>  82
<211>  47
<212>  DNA
<213>  Artificial

<220>
<223>  shRNA

<400>  82
gacgagagaa accttcacct tcaagagagg tgaaggtttc tctcgtc                47


<210>  83
<211>  47
<212>  DNA
<213>  Artificial

<220>
<223>  shRNA

<400>  83
acattattct acattctttt tcaagagaaa agaatgtaga ataatgt                47


<210>  84
<211>  47
<212>  DNA
<213>  Artificial

<220>
<223>  shRNA

<400>  84
agattcgcaa atggatgtat tcaagagata catccatttg cgaatct                47


<210>  85
<211>  47
<212>  DNA
<213>  Artificial

<220>

<223>  shRNA

<400>  85
cattcccacc atgagtctgt tcaagagaca gactcatggt gggaatg                    47


<210>  86
<211>  47
<212>  DNA
<213>  Artificial

<220>
<223>  shRNA

<400>  86
gatcgcccga cacttccgct tcaagagagc ggaagtgtcg ggcgatc                    47


<210>  87
<211>  47
<212>  DNA
<213>  Artificial

<220>
<223>  shRNA

<400>  87
ccagcaggcc tacgtgctgt tcaagagaca gcacgtaggc ctgctgg                    47


<210>  88
<211>  47
<212>  DNA
<213>  Artificial

<220>
<223>  shRNA

<400>  88
gccagctcct ccacagcact tcaagagagt gctgtggagg agctggc                    47


<210>  89
<211>  47
<212>  DNA
<213>  Artificial

<220>
<223>  shRNA

<400>  89
cgccgccaag tggagcagat tcaagagatc tgctccactt ggcggcg                    47


<210>  90
<211>  47
<212>  DNA
<213>  Artificial

<220>
<223>  shRNA

<400>  90

gaagatgccc atgaattcct tcaagagagg aattcatggg catcttc                47

<210>  91
<211>  47
<212>  DNA
<213>  Artificial

<220>
<223>  shRNA

<400>  91
caaacaggct gcgccaggct tcaagagagc ctggcgcagc ctgtttg                47

<210>  92
<211>  47
<212>  DNA
<213>  Artificial

<220>
<223>  shRNA

<400>  92
acggcctagc gcctgatggt tcaagagacc atcaggcgct aggccgt                47

<210>  93
<211>  47
<212>  DNA
<213>  Artificial

<220>
<223>  shRNA

<400>  93
ctgtaacctc tctgatcggt tcaagagacc gatcagagag gttacag                47

<210>  94
<211>  47
<212>  DNA
<213>  Artificial

<220>
<223>  shRNA

<400>  94
tctgtcagtc catcctggct tcaagagagc caggatggac tgacaga                47

<210>  95
<211>  47
<212>  DNA
<213>  Artificial

<220>
<223>  shRNA

<400>  95
tgaagcgaga gtcttgtgat tcaagagatc acaagactct cgcttca                47

<210>  96
<211>  47
<212>  DNA
<213>  Artificial

<220>
<223>  shRNA

<400>  96
gatggagtgc taatggaaat tcaagagatt tccattagca ctccatc                    47


<210>  97
<211>  47
<212>  DNA
<213>  Artificial

<220>
<223>  shRNA

<400>  97
ccttcagaga ttgacacgct tcaagagagc gtgtcaatct ctgaagg                    47


<210>  98
<211>  47
<212>  DNA
<213>  Artificial

<220>
<223>  shRNA

<400>  98
cctgaccacg ttccgactgt tcaagagaca gtcggaacgt ggtcagg                    47


<210>  99
<211>  47
<212>  DNA
<213>  Artificial

<220>
<223>  shRNA

<400>  99
gagttccttc gctgcctgat tcaagagatc aggcagcgaa ggaactc                    47


<210>  100
<211>  47
<212>  DNA
<213>  Artificial

<220>
<223>  shRNA

<400>  100
gactgccttg ctgccttctt tcaagagaag aaggcagcaa ggcagtc                    47


<210>  101
<211>  47
<212>  DNA

<213>  Artificial

<220>
<223>  shRNA

<400>  101
cgccgagggc tacgtactct tcaagagaga gtacgtagcc ctcggcg                    47


<210>  102
<211>  47
<212>  DNA
<213>  Artificial

<220>
<223>  shRNA

<400>  102
ggcgagaaga aaggactgtt tcaagagaac agtcctttct tctcgcc                    47


<210>  103
<211>  47
<212>  DNA
<213>  Artificial

<220>
<223>  shRNA

<400>  103
ggacgagaat tgataaagat tcaagagatc tttatcaatt ctcgtcc                    47


<210>  104
<211>  47
<212>  DNA
<213>  Artificial

<220>
<223>  shRNA

<400>  104
gcacgagaat ttgggaatct tcaagagaga ttcccaaatt ctcgtgc                    47


<210>  105
<211>  47
<212>  DNA
<213>  Artificial

<220>
<223>  shRNA

<400>  105
ctacttcatg aaatattggt tcaagagacc aatatttcat gaagtag                    47


<210>  106
<211>  47
<212>  DNA
<213>  Artificial

<220>

<223>  shRNA

<400>  106
gataacagct tcttgtctat tcaagagata gacaagaagc tgttatc                47


<210>  107
<211>  47
<212>  DNA
<213>  Artificial

<220>
<223>  shRNA

<400>  107
gagaatagga catcagggct tcaagagagc cctgatgtcc tattctc                47


<210>  108
<211>  47
<212>  DNA
<213>  Artificial

<220>
<223>  shRNA

<400>  108
cttggaagac tgaacctgtt tcaagagaac aggttcagtc ttccaag                47


<210>  109
<211>  47
<212>  DNA
<213>  Artificial

<220>
<223>  shRNA

<400>  109
caactccttt gtggatgcat tcaagagatg catccacaaa ggagttg                47


<210>  110
<211>  47
<212>  DNA
<213>  Artificial

<220>
<223>  shRNA

<400>  110
gatgttgtct ccaaatgcat tcaagagatg catttggaga caacatc                47


<210>  111
<211>  47
<212>  DNA
<213>  Artificial

<220>
<223>  shRNA

<400>  111

cgtggggact gtacctccct tcaagagagg gaggtacagt ccccacg                47

<210>  112
<211>  47
<212>  DNA
<213>  Artificial

<220>
<223>  shRNA

<400>  112
gtacagcttc agaaccaagt tcaagagact tggttctgaa gctgtac                47

<210>  113
<211>  47
<212>  DNA
<213>  Artificial

<220>
<223>  shRNA

<400>  113
gatgatcttc agagagcaat tcaagagatt gctctctgaa gatcatc                47

<210>  114
<211>  47
<212>  DNA
<213>  Artificial

<220>
<223>  shRNA

<400>  114
ggaacatcgg aatttgcctt tcaagagaag gcaaattccg atgttcc                47

<210>  115
<211>  47
<212>  DNA
<213>  Artificial

<220>
<223>  shRNA

<400>  115
gagctagtga gggactcttt tcaagagaaa gagtccctca ctagctc                47

<210>  116
<211>  47
<212>  DNA
<213>  Artificial

<220>
<223>  shRNA

<400>  116
gcagggttct ttaaggcaat tcaagagatt gccttaaaga accctgc                47

<210>  117
<211>  47
<212>  DNA
<213>  Artificial

<220>
<223>  shRNA

<400>  117
tcgatgattc ctctgaaact tcaagagagt ttcagaggaa tcatcga                47


<210>  118
<211>  47
<212>  DNA
<213>  Artificial

<220>
<223>  shRNA

<400>  118
gataatggaa atattgaact tcaagagagt tcaatatttc cattatc                47


<210>  119
<211>  47
<212>  DNA
<213>  Artificial

<220>
<223>  shRNA

<400>  119
gttcttcatt taaatgatat tcaagagata tcatttaaat gaagaac                47


<210>  120
<211>  47
<212>  DNA
<213>  Artificial

<220>
<223>  shRNA

<400>  120
gttaacaaac acataaagtt tcaagagaac tttatgtgtt tgttaac                47


<210>  121
<211>  47
<212>  DNA
<213>  Artificial

<220>
<223>  shRNA

<400>  121
gttagagaag attcttcgtt tcaagagaac gaagaatctt ctctaac                47


<210>  122
<211>  47
<212>  DNA

<213>  Artificial

<220>
<223>  shRNA

<400>  122
gttgattgga caattaaact tcaagagagt ttaattgtcc aatcaac                    47


<210>  123
<211>  47
<212>  DNA
<213>  Artificial

<220>
<223>  shRNA

<400>  123
ggttgatacc gtaaagcgct tcaagagagc gctttacggt atcaacc                    47


<210>  124
<211>  47
<212>  DNA
<213>  Artificial

<220>
<223>  shRNA

<400>  124
gcaatgaaac gtccaatggt tcaagagacc attggacgtt tcattgc                    47


<210>  125
<211>  47
<212>  DNA
<213>  Artificial

<220>
<223>  shRNA

<400>  125
agctagagaa aattcttcgt tcaagagacg aagaattttc tctagct                    47


<210>  126
<211>  47
<212>  DNA
<213>  Artificial

<220>
<223>  shRNA

<400>  126
gatcctatat gatggatgat tcaagagatc atccatcata taggatc                    47


<210>  127
<211>  47
<212>  DNA
<213>  Artificial

<220>

<223>  shRNA

<400>  127
gttcttcttg tcagtgaaat tcaagagatt tcactgacaa gaagaac                    47


<210>  128
<211>  47
<212>  DNA
<213>  Artificial

<220>
<223>  shRNA

<400>  128
cttgagcttg agtgaccact tcaagagagt ggtcactcaa gctcaag                    47


<210>  129
<211>  47
<212>  DNA
<213>  Artificial

<220>
<223>  shRNA

<400>  129
gaccggccag cgagtctact tcaagagagt agactcgctg gccggtc                    47


<210>  130
<211>  47
<212>  DNA
<213>  Artificial

<220>
<223>  shRNA

<400>  130
ggacctgggc tacatctact tcaagagagt agatgtagcc caggtcc                    47


<210>  131
<211>  47
<212>  DNA
<213>  Artificial

<220>
<223>  shRNA

<400>  131
ctctgtggtc caggtgctct tcaagagaga gcacctggac cacagag                    47


<210>  132
<211>  47
<212>  DNA
<213>  Artificial

<220>
<223>  shRNA

<400>  132

gaccacacga tttgcctcat tcaagagatg aggcaaatcg tgtggtc                47

<210>  133
<211>  47
<212>  DNA
<213>  Artificial

<220>
<223>  shRNA

<400>  133
tggcttgttt attgaaggat tcaagagatc cttcaataaa caagcca                47

<210>  134
<211>  47
<212>  DNA
<213>  Artificial

<220>
<223>  shRNA

<400>  134
gtgaatttgg ggaagataat tcaagagatt atcttcccca aattcac                47

<210>  135
<211>  47
<212>  DNA
<213>  Artificial

<220>
<223>  shRNA

<400>  135
cgctatagct tgaatgagtt tcaagagaac tcattcaagc tatagcg                47

<210>  136
<211>  47
<212>  DNA
<213>  Artificial

<220>
<223>  shRNA

<400>  136
gatatcctgg ctccacacat tcaagagatg tgtggagcca ggatatc                47

<210>  137
<211>  47
<212>  DNA
<213>  Artificial

<220>
<223>  shRNA

<400>  137
gagccagtcg gatgtagatt tcaagagaat ctacatccga ctggctc                47

<210>  138
<211>  47
<212>  DNA
<213>  Artificial

<220>
<223>  shRNA

<400>  138
gtaaattctg aaggcgaatt tcaagagaat tcgccttcag aatttac                    47


<210>  139
<211>  47
<212>  DNA
<213>  Artificial

<220>
<223>  shRNA

<400>  139
gccctcctaa atcaggcaat tcaagagatt gcctgattta ggagggc                    47


<210>  140
<211>  47
<212>  DNA
<213>  Artificial

<220>
<223>  shRNA

<400>  140
gttgagaaat ggagtgaagt tcaagagact tcactccatt tctcaac                    47


<210>  141
<211>  47
<212>  DNA
<213>  Artificial

<220>
<223>  shRNA

<400>  141
gcttggaaaa tgcaaggcgt tcaagagacg ccttgcattt tccaagc                    47


<210>  142
<211>  47
<212>  DNA
<213>  Artificial

<220>
<223>  shRNA

<400>  142
ctgcatcata gaccagatct tcaagagaga tctggtctat gatgcag                    47


<210>  143
<211>  47
<212>  DNA

<213>  Artificial

<220>
<223>  shRNA

<400>  143
gatcaccacg tatgtgtcct tcaagagagg acacatacgt ggtgatc                47


<210>  144
<211>  47
<212>  DNA
<213>  Artificial

<220>
<223>  shRNA

<400>  144
tgacaacaag tattccctgt tcaagagaca gggaatactt gttgtca                47


<210>  145
<211>  47
<212>  DNA
<213>  Artificial

<220>
<223>  shRNA

<400>  145
gaaatataag acagattcct tcaagagagg aatctgtctt atatttc                47


<210>  146
<211>  47
<212>  DNA
<213>  Artificial

<220>
<223>  shRNA

<400>  146
cccatcaagt ttagaggatt tcaagagaat cctctaaact tgatggg                47


<210>  147
<211>  47
<212>  DNA
<213>  Artificial

<220>
<223>  shRNA

<400>  147
ggtgtcccat gggaatatat tcaagagata tattcccatg ggacacc                47


<210>  148
<211>  47
<212>  DNA
<213>  Artificial

<220>

<223>  shRNA

<400>  148
gaatgccgac ctacaaagat tcaagagatc tttgtaggtc ggcattc                47


<210>  149
<211>  47
<212>  DNA
<213>  Artificial

<220>
<223>  shRNA

<400>  149
cagttatatt ctgtgatgtt tcaagagaac atcacagaat ataactg                47


<210>  150
<211>  47
<212>  DNA
<213>  Artificial

<220>
<223>  shRNA

<400>  150
gaggtgttgg ggacaaaggt tcaagagacc tttgtcccca acacctc                47


<210>  151
<211>  47
<212>  DNA
<213>  Artificial

<220>
<223>  shRNA

<400>  151
gtgggctcat tggctgaagt tcaagagact tcagccaatg agcccac                47


<210>  152
<211>  47
<212>  DNA
<213>  Artificial

<220>
<223>  shRNA

<400>  152
gagctactga ggacagaaat tcaagagatt tctgtcctca gtagctc                47


<210>  153
<211>  47
<212>  DNA
<213>  Artificial

<220>
<223>  shRNA

<400>  153

tcagcaggat gctcaggagt tcaagagact cctgagcatc ctgctga                47

<210>  154
<211>  47
<212>  DNA
<213>  Artificial

<220>
<223>  shRNA

<400>  154
gaagttctcc atccagaggt tcaagagacc tctggatgga gaacttc                47

<210>  155
<211>  47
<212>  DNA
<213>  Artificial

<220>
<223>  shRNA

<400>  155
gccggtcccc accagcagct tcaagagagc tgctggtggg gaccggc                47

<210>  156
<211>  47
<212>  DNA
<213>  Artificial

<220>
<223>  shRNA

<400>  156
cactcgggag ttgagagatt tcaagagaat ctctcaactc ccgagtg                47

<210>  157
<211>  47
<212>  DNA
<213>  Artificial

<220>
<223>  shRNA

<400>  157
gcccttgggt ctgtttgact tcaagagagt caaacagacc caagggc                47

<210>  158
<211>  47
<212>  DNA
<213>  Artificial

<220>
<223>  shRNA

<400>  158
ctcaacacta aacagcaagt tcaagagact tgctgtttag tgttgag                47

<210>  159
<211>  47
<212>  DNA
<213>  Artificial

<220>
<223>  shRNA

<400>  159
gatttcattg gacagcatat tcaagagata tgctgtccaa tgaaatc                    47


<210>  160
<211>  47
<212>  DNA
<213>  Artificial

<220>
<223>  shRNA

<400>  160
catggggcac caactaattt tcaagagaaa ttagttggtg ccccatg                    47


<210>  161
<211>  47
<212>  DNA
<213>  Artificial

<220>
<223>  shRNA

<400>  161
ggtgtctctg cgggattgtt tcaagagaac aatcccgcag agacacc                    47


<210>  162
<211>  47
<212>  DNA
<213>  Artificial

<220>
<223>  shRNA

<400>  162
agttcagtag gtgtagactt tcaagagaag tctacaccta ctgaact                    47


<210>  163
<211>  47
<212>  DNA
<213>  Artificial

<220>
<223>  shRNA

<400>  163
gagttcctga agctcctcat tcaagagatg aggagcttca ggaactc                    47


<210>  164
<211>  47
<212>  DNA

<213>  Artificial

<220>
<223>  shRNA

<400>  164
ggatttgctg ggggcaaggt tcaagagacc ttgcccccag caaatcc                    47


<210>  165
<211>  47
<212>  DNA
<213>  Artificial

<220>
<223>  shRNA

<400>  165
ctcagaaagc caacattcat tcaagagatg aatgttggct ttctgag                    47


<210>  166
<211>  47
<212>  DNA
<213>  Artificial

<220>
<223>  shRNA

<400>  166
cgcattgtaa taagaaggtt tcaagagaac cttcttatta caatgcg                    47


<210>  167
<211>  47
<212>  DNA
<213>  Artificial

<220>
<223>  shRNA

<400>  167
gggaggaaaa tgcagaaatt tcaagagaat ttctgcattt tcctccc                    47


<210>  168
<211>  47
<212>  DNA
<213>  Artificial

<220>
<223>  shRNA

<400>  168
ttacaaattt aggaaatact tcaagagagt atttcctaaa tttgtaa                    47


<210>  169
<211>  47
<212>  DNA
<213>  Artificial

<220>

<223>  shRNA

<400>  169
gttatgaatt gatatgcagt tcaagagact gcatatcaat tcataac                47


<210>  170
<211>  47
<212>  DNA
<213>  Artificial

<220>
<223>  shRNA

<400>  170
gtgataacac aactaatggt tcaagagacc attagttgtg ttatcac                47


<210>  171
<211>  47
<212>  DNA
<213>  Artificial

<220>
<223>  shRNA

<400>  171
gtagaggaga gttctgaaat tcaagagatt tcagaactct cctctac                47


<210>  172
<211>  47
<212>  DNA
<213>  Artificial

<220>
<223>  shRNA

<400>  172
gcctctaatc ctgataaggt tcaagagacc ttatcaggat tagaggc                47


<210>  173
<211>  47
<212>  DNA
<213>  Artificial

<220>
<223>  shRNA

<400>  173
gatgatcttc aggctgccat tcaagagatg gcagcctgaa gatcatc                47


<210>  174
<211>  47
<212>  DNA
<213>  Artificial

<220>
<223>  shRNA

<400>  174

gtatggacaa gagcgttggt tcaagagacc aacgctcttg tccatac                47

<210>  175
<211>  47
<212>  DNA
<213>  Artificial

<220>
<223>  shRNA

<400>  175
cgaacccttc tggaacagtt tcaagagaac tgttccagaa gggttcg                47

<210>  176
<211>  47
<212>  DNA
<213>  Artificial

<220>
<223>  shRNA

<400>  176
gtggcatgaa gattatagtt tcaagagaac tataatcttc atgccac                47

<210>  177
<211>  47
<212>  DNA
<213>  Artificial

<220>
<223>  shRNA

<400>  177
ggtgaacaag gacagtatct tcaagagaga tactgtcctt gttcacc                47

<210>  178
<211>  47
<212>  DNA
<213>  Artificial

<220>
<223>  shRNA

<400>  178
gcaatagagg atgattctgt tcaagagaca gaatcatcct ctattgc                47

<210>  179
<211>  47
<212>  DNA
<213>  Artificial

<220>
<223>  shRNA

<400>  179
tctgtgaatg ccaaagttct tcaagagaga actttggcat tcacaga                47

<210>  180
<211>  47
<212>  DNA
<213>  Artificial

<220>
<223>  shRNA

<400>  180
cacaccaggg aaggtctagt tcaagagact agaccttccc tggtgtg                47


<210>  181
<211>  47
<212>  DNA
<213>  Artificial

<220>
<223>  shRNA

<400>  181
gcaggaagat gcccatgaat tcaagagatt catgggcatc ttcctgc                47


<210>  182
<211>  47
<212>  DNA
<213>  Artificial

<220>
<223>  shRNA

<400>  182
gaatgtgcaa tatcctgagt tcaagagact caggatattg cacattc                47


<210>  183
<211>  47
<212>  DNA
<213>  Artificial

<220>
<223>  shRNA

<400>  183
tggatgatgc caaggtcact tcaagagagt gaccttggca tcatcca                47


<210>  184
<211>  47
<212>  DNA
<213>  Artificial

<220>
<223>  shRNA

<400>  184
gctccgtgct aaacctctct tcaagagaga gaggtttagc acggagc                47


<210>  185
<211>  47
<212>  DNA

<213>  Artificial

<220>
<223>  shRNA

<400>  185
gcctccacct caacagaggt tcaagagacc tctgttgagg tggaggc                    47


<210>  186
<211>  47
<212>  DNA
<213>  Artificial

<220>
<223>  shRNA

<400>  186
ctgcatcata gaccaaatct tcaagagaga tttggtctat gatgcag                    47


<210>  187
<211>  47
<212>  DNA
<213>  Artificial

<220>
<223>  shRNA

<400>  187
gatcactaca tacatttcct tcaagagagg aaatgtatgt agtgatc                    47


<210>  188
<211>  47
<212>  DNA
<213>  Artificial

<220>
<223>  shRNA

<400>  188
gtaaagagag cagaatgaat tcaagagatt cattctgctc tctttac                    47


<210>  189
<211>  47
<212>  DNA
<213>  Artificial

<220>
<223>  shRNA

<400>  189
cgcggggcgc agtggtatct tcaagagaga taccactgcg ccccgcg                    47


<210>  190
<211>  47
<212>  DNA
<213>  Artificial

<220>

<223>  shRNA

<400>  190
cagaaggcag tggggaagat tcaagagatc ttccccactg ccttctg                    47


<210>  191
<211>  47
<212>  DNA
<213>  Artificial

<220>
<223>  shRNA

<400>  191
gcctgggaga atcacaggtt tcaagagaac ctgtgattct cccaggc                    47


<210>  192
<211>  47
<212>  DNA
<213>  Artificial

<220>
<223>  shRNA

<400>  192
accagacaag gaaataccct tcaagagagg gtatttcctt gtctggt                    47


<210>  193
<211>  47
<212>  DNA
<213>  Artificial

<220>
<223>  shRNA

<400>  193
cacatccacc acatcgacct tcaagagagg tcgatgtggt ggatgtg                    47


<210>  194
<211>  47
<212>  DNA
<213>  Artificial

<220>
<223>  shRNA

<400>  194
gtcacaaccc aagaccatgt tcaagagaca tggtcttggg ttgtgac                    47


<210>  195
<211>  47
<212>  DNA
<213>  Artificial

<220>
<223>  shRNA

<400>  195

ctcaacagga caaatcccat tcaagagatg ggatttgtcc tgttgag 47

<210> 196
<211> 47
<212> DNA
<213> Artificial

<220>
<223> shRNA

<400> 196
tagatcaatt attgtggatt tcaagagaat ccacaataat tgatcta 47

<210> 197
<211> 47
<212> DNA
<213> Artificial

<220>
<223> shRNA

<400> 197
ggaacacctt attgatgaat tcaagagatt catcaataag gtgttcc 47

<210> 198
<211> 47
<212> DNA
<213> Artificial

<220>
<223> shRNA

<400> 198
ctttaacaga aattgtctct tcaagagaga gacaatttct gttaaag 47

<210> 199
<211> 47
<212> DNA
<213> Artificial

<220>
<223> shRNA

<400> 199
cctatgcagt acaaagtggt tcaagagacc actttgtact gcatagg 47

<210> 200
<211> 47
<212> DNA
<213> Artificial

<220>
<223> shRNA

<400> 200
gatcttttct tgctttggat tcaagagatc caaagcaaga aaagatc 47

<210>  201
<211>  47
<212>  DNA
<213>  Artificial

<220>
<223>  shRNA

<400>  201
cagcatcctt caggccttat tcaagagata aggcctgaag gatgctg                47


<210>  202
<211>  47
<212>  DNA
<213>  Artificial

<220>
<223>  shRNA

<400>  202
gatagtgact cggatctgct tcaagagagc agatccgagt cactatc                47


<210>  203
<211>  47
<212>  DNA
<213>  Artificial

<220>
<223>  shRNA

<400>  203
gacatcacag cccgggagtt tcaagagaac tcccgggctg tgatgtc                47


<210>  204
<211>  47
<212>  DNA
<213>  Artificial

<220>
<223>  shRNA

<400>  204
ggacacagcc tatgtgctgt tcaagagaca gcacataggc tgtgtcc                47


<210>  205
<211>  47
<212>  DNA
<213>  Artificial

<220>
<223>  shRNA

<400>  205
gtggaggaga tctacgacct tcaagagagg tcgtagatct cctccac                47


<210>  206
<211>  47
<212>  DNA

<213>  Artificial

<220>
<223>  shRNA

<400>  206
ctcttgtgca actcatgcct tcaagagagg catgagttgc acaagag                47


<210>  207
<211>  47
<212>  DNA
<213>  Artificial

<220>
<223>  shRNA

<400>  207
acagggcccc tgcagcctct tcaagagaga ggctgcaggg gccctgt                47


<210>  208
<211>  47
<212>  DNA
<213>  Artificial

<220>
<223>  shRNA

<400>  208
gaagacctgg cggcaggtgt tcaagagaca cctgccgcca ggtcttc                47


<210>  209
<211>  21
<212>  DNA
<213>  Artificial

<220>
<223>  siRNA

<400>  209
gcctgtgcct cttcagctac c                                            21


<210>  210
<211>  21
<212>  DNA
<213>  Artificial

<220>
<223>  siRNA

<400>  210
gcggagacag cgacgaagag c                                            21


<210>  211
<211>  19
<212>  DNA
<213>  Artificial

<220>

<223>  siRNA

<400>  211
cttattggag agagcacga                                                    19

## Description

The invention provides particular lentiviral vectors that are useful for the transfection of cells with shRNAs. Moreover, cells transfected with such vectors and non-human vertebrates having such cells are provided.

### Background of the Invention

Animal models are powerful tools for studying gene functions in vivo. Through ubiquitous or tissue-specific over-expression of protein-coding cDNAs, the genome of rodents can be manipulated. In addition it is possible in mice to inactivate certain gene products by homologous recombination in embryonic stem cells (Glaser, S. et al., Nat. Genet. 37(11):1187-1193 (2005)). This technique however has the disadvantage that it is (i) only applicable in mice, (ii) very time and cost intensive and (iii) not reversible. In many experimental contexts, such as mimicking effects of potential targeted therapeutics in models of human disease, other animal models than the mouse and transient inhibition of endogenous genes would be desirable (Benson, J. D. et al., Nature 441(7092):451-456 (2006)).

RNA interference (RNAi) holds great promise as an experimental tool for loss-of-function genetics. This technology is based on the introduction of short double-stranded RNAs - also called small interfering RNAs (siRNAs) - into cells, where one strand shows sequence homology to a specific gene transcript. Due to the binding of the siRNA to its target, the siRNA:mRNA complex is incorporated into a large enzyme complex called RNA induced silencing complex (RISC) leading to the degradation of the messenger RNA and knock-down of the gene (Fire, A. et al., Nature 391(6669):806-811 (1998); Sontheimer, E. J., Nat. Rev. Mol. Cell. Biol. 6(2):127-138 (2005)). One possibility to generate those siRNAs in cells is the expression of a precursor RNA, called small hairpin RNA (shRNA). Transcription of shRNAs is usually driven by RNA-polymerase-III promoters like the U6 or H1 promoter (Tuschl, T., Nat. Biotechnol. 20(5):446-448 (2002); Brummelkamp, T. R. et al., Science 296(5567):550-553 (2002)). Incorporation of such an shRNA cassette into viral vector constructs for example makes it suitable for transient or stable transfection and knock-down of certain gene products *in vitro* as well as *in vivo* (Tiscornia, G. et al., Proc. Natl. Acad. Sci. U S A 100(4):1844-1848 (2003); Rubinson, D. A. et al., Nat. Genet. 33(3):401-406 (2003)).

Lentviruses have the unique feature to infect not only dividing cells, but also non dividing cells of nearly all species. Therefore those vectors have been employed for the generation of different transgenic models like mice, rats, pig and cattle (Pfeifer, A., Curr. Gene Ther. 6(4):535-542 (2006)). Initially they were used for the overexpression of protein coding cDNAs. Recently however, RNAi technology and lentiviruses were combined as a tool for a gene specific knock-down in transgenic mouse models (Tiscornia, G. et al., Proc. Natl. Acad. Sci. U S A 100(4):1844-1848 (2003); Rubinson, D. A. et al., Nat. Genet. 33(3):401-406 (2003)). In addition lentiviral knock-down technology was also used in a rat model for the constitutive inhibition of Dazl, a gene expressed in germ cells and required for fertility (Dann, C. T. et al., Proc. Natl. Acad. Sci. U S A 103(30):11246-11251 (2006)).

Moreover, lentiviral transfer plasmids were also used for transfection of eukaryotic cells with siRNAs or shRNAs (WO2004/022722 US-A-20050251872; WO2007/109131).

Inducible expression of shRNAs has been achieved *in vitro* by using RNA polymerase III-dependent promoters containing operator sequences (tetO) of the *E*. *coli* tetracycline resistance operon (Czauderna, F. et al., Nucleic Acids Res. 31(21):e127 (2003); Matsukura, S. et al., Nucleic Acids Res. 31(15):e77 (2003); van de Wetering et al., EMBO Rep. 4(6):609-615 (2003); Ohkawa, J. et al., Hum. Gene Ther. 11(4):577-585 (2000)). The tetracycline resistance operon repressor (tetR) can bind to tetO and thereby block transcription. Addition of doxycycline leads to de-repression of the transcriptional block by releasing the tetR. Recently, a mouse model for the tight control of RNAi was established (Seibler, J. et al., Nucleic Acids Res. 35(7):e54 (2007); WO 06/131543), where the vector constructs were integrated at a well defined loci by means of homologous recombination. The utility of said vector construct is however limited and the integration procedure is quite limiting.

### Summary of the Invention

It has now been found that Lentiviral vectors are well suited for the expression of shRNAs for the inhibition of gene products in cell lines as well as in transgenic animals. As the lentiviral transfection vector requires the presence of a marker molecule, it was found that the marker molecule is advantageously integrated downstream of the codon-optimized repressor gene of the regulator construct and connected with the latter via a viral peptide sequence. Initially this leads to the generation of a bicistronic mRNA, containing tetR and GFP. Due to the viral sequence however, the ribosomes do not form a peptide bond between the C-terminus of the tetR and the N-terminus of the GFP, resulting in two independent proteins. The invention thus provides
(1) an inducible lentiviral vector comprising
   (a) a responder construct comprising at least one segment corresponding to a short hairpin RNA (shRNA) or to complementary short interfering RNA (siRNA) strands, said segment being under control of a ubiquitous promoter, wherein said promoter contains at least one operator sequence, by which said promoter is perfectly and ubiquitously regulatable by a repressor and
   (b) a regulator construct comprising a codon-optimized repressor gene, which provides for perfect regulation of the promoter of the responder construct, said codon-optimized repressor gene is located upstream of a marker gene and is under the control of a ubiquitously active promoter, and said codon-optimized repressor gene and said marker gene being linked by a DNA sequence encoding a viral peptide sequence;
(2) a preferred embodiment of (1) above, wherein the responder construct is a H1-tetO-shRNA cassette, preferably having the sequence of bp 2594 to 2953 of SEQ ID NO:1 and/or the a regulator construct comprises a codon optimized Tet-Repressor located upstream of the eGFP marker gene and is under the control of the ubiquitin promoter, and said TetR and said marker gene being linked by the viral T2A peptide sequence, preferably said regulator construct has the sequence of bp 2974 to 5609 of SEQ ID NO:1;
(3) a biological entity selected from a non-human vertebrate, a tissue culture derived from a vertebrate or one or more cells of a cell culture derived from a vertebrate, said biological entity carrying a responder construct and a regulator construct as defined in (1) or (2) above, wherein the responder construct and/or the regulator construct are stably integrated into the genome of the biological entity, preferably at actively transcribed regions;
(4) a method for preparing the biological entity as defined in (3) above or a method for inducible gene knock down in a biological entity, which method comprises stably integrating a responder construct and a regulator construct into the genome of the biological entity by viral transduction with a lentiviral vector as defined in (1) or (2) above; and
(5) the use of a biological entity as defined in (3) above inducible gene knock down, and/or as a test system for pharmaceutical testing, and/or for gene target validation, and/or for gene function analysis.

### Short Description of the Figures

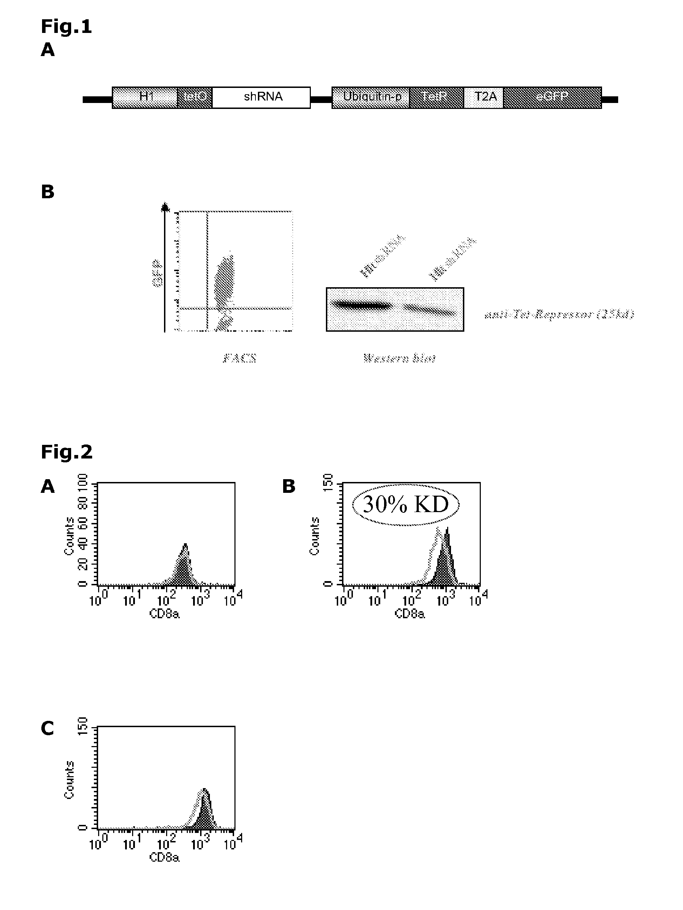

Figure 1: Design and test of the shRNA inducible vector in vitro. Schematic drawing of the inducible lentiviral vector construct (A). Hybridoma cells were transduced with FH1tshRNA-UTG. Five days after transduction cells were analyzed for eGFP and the Tet-repressor protein by flow cytometrie and western blot respectively. For western blotting two FH1tshRNA-UTG transduced hybridoma cell populations were analyzed and probed with the anti-tetR antibody.
Figure 2: Test of shRNA induction and reversibility in vitro. Hybridoma cells were transduced with FH1tshCD8aUTG. Transduced (-) versus non transduced (■) cells were stained for the surface expression of CD8-alpha in media alone (A), after 7 days of culturing in 1 µg/ml dox media (B) and after 7 days in 1 µg/ml dox media plus another 10 days of media alone (C) and subsequently analyzed by flow cytometry.

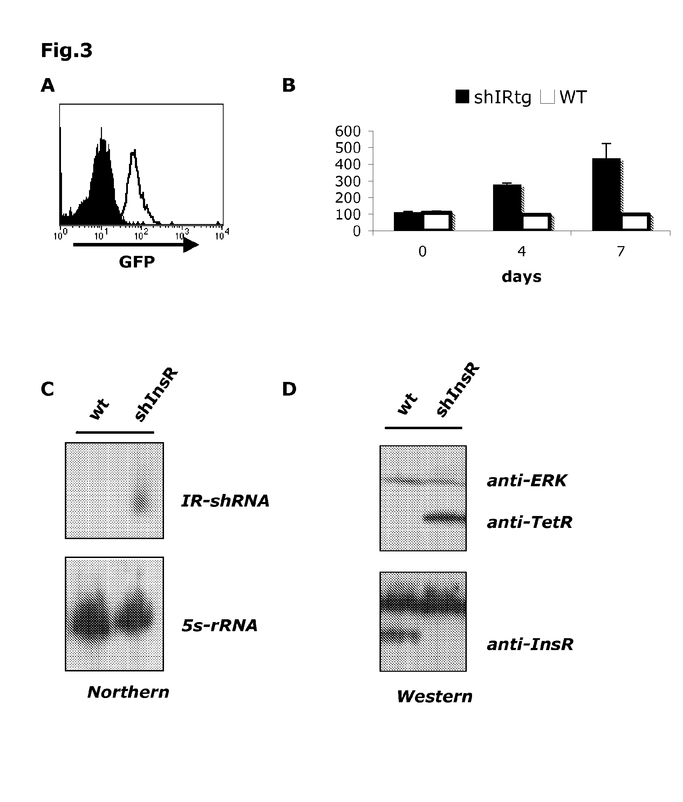

Figure 3: Induction of shRNA targeting the Insulin receptor in rats leads to signs of diabetes. Flow cytometric analysis of FH1tIR5UTG tg (-) versus non-tg (■) rats based on eGFP expression (A). Rats were fed for seven days with water containing 2 mg/ml of dox and blood glucose levels analyzed at the indicated time points. Day 0 is the starting point of dox administration. (B). Northern Blot analysis of RNA obtained from liver of tg rats and wt rats after administration of 2 mg/ml dox in the drinking water for seven days (C). Western Blot analysis from liver proteins obtained from rats treated with 2 mg/ml dox for seven days. Upper Blot shows the expression of the tetR and lower blot of the InsR (D).

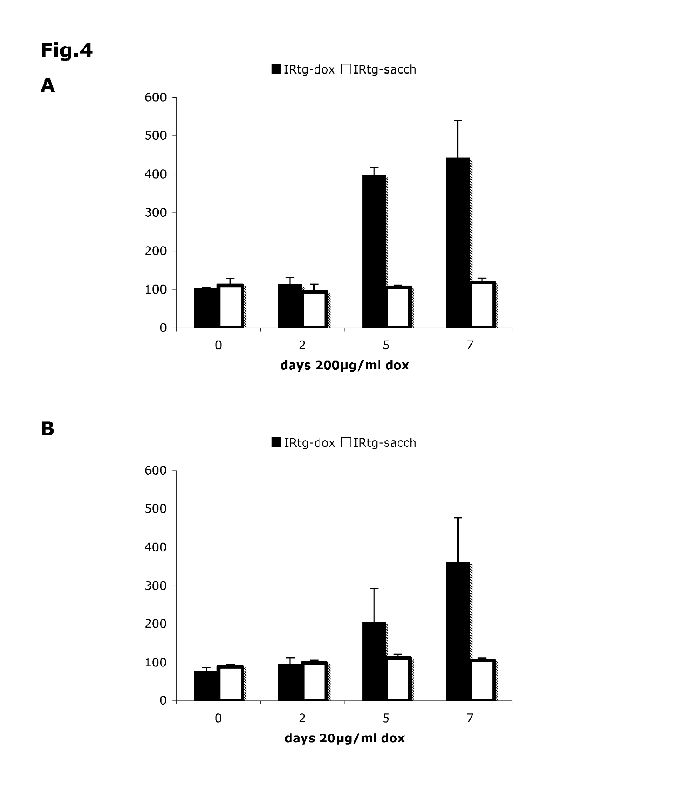

Figure 4: Induction of high blood glucose levels occurs at very low dox concentrations. FH1tIRSUTG tg Rats were fed for seven days with water containing 200 µg/ml dox (■) (A) or 20 µg/ml dox (■) (B) water containing only saccharose (□).Blood glucose levels were monitored at the indicated time points. Day 0 is the starting point of dox administration.

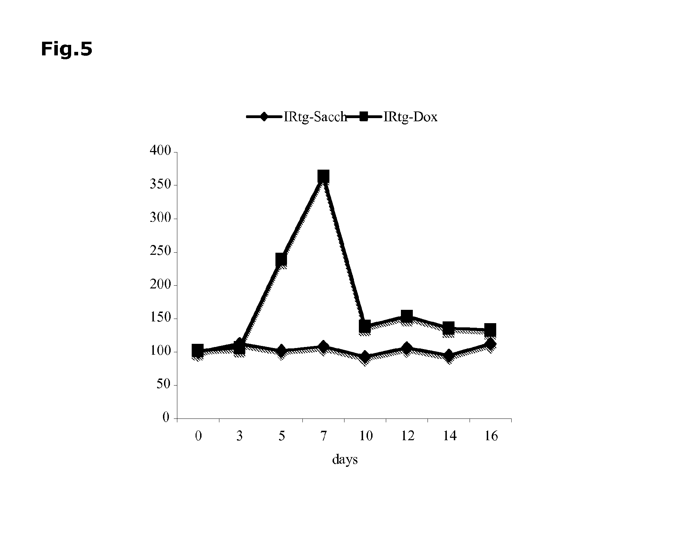

Figure 5: Signs of type II diabetes can be reversed through withdrawal of doxycycline. FH1tIR5UTG tg Rats were fed for seven days with water containing 20 µg/ml dox (■) or water containing only saccharose (◆). Afterwards rats obtained pure water. Blood glucose levels were monitored at the indicated time points. Day 0 is the starting point of dox administration.

### Detailed description of the Invention

The invention described hereinbefore provides a tool for the stable transduction of cell lines with a single vector allowing the inducible expression of a shRNA. The "lentiviral vector" of aspects (1) and (2) of the invention is an integration construct that contains all the necessary sequences for packaging into viral particles and its stable integration into the genome.

The "biological entity" according to the present invention includes, but is not limited to, a vertebrate, a tissue culture derived from a vertebrate, or one or more cells of a cell culture derived from a vertebrate.

The term "vertebrate" according to the present invention relates to multicellular organisms such as mammals, e.g. non-human animals such as rodents (including mice, rats, etc.) and humans, or non-mammals, e.g. fish. Most preferred vertebrates are mice, rats and fish.

"Tissue culture" according to the present invention refers to parts of the above-defined "vertebrates" (including organs and the like) which are cultured *in vitro.*

"Cell culture" according to the present invention includes cells isolated from the above-defined "vertebrates" which are cultured *in vitro.* These cells can be transformed (immortalized) or untransformed (directly derived from vertebrates; primary cell culture).

The term "living organisms" according to the present invention relates to multi-cell organisms, which can be vertebrates such as mammals (e.g. non-human animals such as rodents including mice and rats; and humans) or non-mammals (e.g. fish) or can be invertebrates such as insects or worms, or can be plants (higher plants, algi or fungi). Most preferred living organisms are mice, rats and fish.

"Eukaryotic cells" and "starting eukaryotic cells" according to the present invention include cells isolated (derived) from the above-defined living organisms and cultured *in vitro.* These cells can be transformed (immortalized) or untransformed (directly derived from living organisms; primary cell culture). The term "eukaryotic cells" also includes mono-cellular eukaryotic cells such as yeasts, etc.

In the lentiviral vector of aspect (1) of the invention the responder construct and the regulator construct allow inducible gene knock down in said biological entity. Notably, the regulation by said repressor permits control of the expression and the suppression of the expression of the shRNA or the siRNA by a rate of at least 90%, preferably by a rate of at least 95%, more preferably by a rate of at least 98%, and most preferably by a rate of 100%.

The promoter of the responder construct is selected from RNA polymerase (Pol) I, II and III dependent promoters, preferably is a Pol II and III dependent promoter, more preferably is selected from a CMV promoter, a CAGGS promoter, a Ubiquitin promoter, a RNAse P RNA promoter such as H1, a tRNA promoter, a 7SL RNA promoter, and a 5 S rRNA promoter.

The promoter of the regulator construct is selected from RNA polymerase (Pol) I, II and III dependent promoters, preferably is a Pol II and III dependent promoter, more preferably is selected from a CMV promoter, a CAGGS promoter, a Ubiquitin promoter, a snRNA promoter such as U6, a RNAse P RNA promoter such as H1, a tRNA promoter, a 7SL RNA promoter, and a 5 S rRNA promoter.

The responder construct and/or the regulator construct may further contain functional sequences selected from splice acceptor sequences, selectable marker sequences, recognition sequences, etc.

The DNA sequence encoding a viral peptide sequence of the regulator construct is selected from viral sequences from foot-and-mouth disease virus F2A (including that of SEQ ID NO:5), equine rhinitis A virus E2A (including that of SEQ ID NO:6), porcine teschovirus-1 P2A and Thosea asigna viral T2A (including that of bp 4836 to 4889 of SEQ ID NO:1), and most preferably is the Thosea asigna viral T2A peptide sequence having the sequence of bp 4836 to 4889 of SEQ ID NO:1.

The lentiviral vector of the invention is suitable to be stably integrated into the genome of a biological entity, preferably at actively transcribed regions.

In a particularly preferred embodiment of aspect (1) of the invention, the promoter of the responder construct is an inducible promoter selected from polymerase (Pol) III dependent promoters, preferably is an RNAse P RNA promoter such as H1, and most preferably is a H1-promoter.

It is furthermore preferred that the promoter of the responder construct contains an operator sequence selected from tetO, GalO, lacO, etc., preferably the operator sequence is tetO. It is also preferred embodiment that the operator sequence of the promoter is positioned 1 to 10 bp, preferably1 to 2 bp 3' (i.e., downstream) and/or 5' (i.e., upstream) of the TATA element. It is moreover preferred that the DNA sequence corresponding to the shRNA or siRNA is positioned 3' to said operator sequence.

In a particularly preferred embodiment of aspect (1) of the invention the responder construct carries a Pol III dependent promoter, preferably an inducible H1 promoter, containing the operator, such as tetO, and the segment(s) corresponding to a shRNA or siRNA.

The responder construct may further comprise a stop and/or a polyadenylation sequence.

It is particularly preferred that the responder construct according to embodiment (1) of the invention comprises an shRNA sequence, i.e. a short hairpin RNA (shRNA) or to complementary short interfering RNA (siRNA) strands. In case shRNA segments are utilized within the expression construct, said construct preferably comprises at least one shRNA segment having a nucleotide (e.g. DNA) sequence of the structure A-B-C or C-B-A. In case siRNA segments are utilized within the expression construct, said cassette preferably comprises at least two DNA segments A and C or C and A, wherein each of said at least two segments is under the control of a separate promoter as defined above (such as the Pol III promoter including inducible U6, H1 or the like). In the above segments
A is a 15 to 35, preferably a 19 to 29 bp DNA sequence being at least 90%, preferably 100% complementary to the gene to be knocked down (e.g. firefly luciferase, p53, etc.);
B is a spacer DNA sequence having 5 to 9 bp forming the loop of the expressed RNA hairpin molecule, and
C is a 15 to 35, preferably a 19 to 29 bp DNA sequence being at least 85% complementary to the sequence A.

The above shRNA and siRNA segments may further comprise stop and/or polyadenylation sequences.

Suitable shRNA sequences for the knock down of a given target gene are well known in the art (see e.g. the particular shRNA sequences mentioned in Tables 1 and 2 below) or can readily be determined by aperson skilled in the art.

**Table 1:**

| target gene | shRNA sequence (SEQ ID NO) | Reference |
|---|---|---|
| CDH-1 p53 CDC20 | | Brummelkamp et al., Science, 296: 550-3 (2002). |
| CYLD | CctcatgcagttctctttgTTCAAGAGAcaaagagaactgcatgagg (10) | Kovalenko et al, Nature, 424: 801-5 (2003). |
| Ras-Gap | AagatgaagccactccctatttCAAGAGAaaatagggagtggcttcatctt (11) | Kunath et al., Nature Biotech, 21: 559-561 (2003) |
| tubulin | GacagagccaagtggactcACAgagtccacttggctctgtc (12) | Yu et al., PNAS, 99: 6047-52 (2002) |
| lamin | Ctggacttccagaagaacattcgtgttcttctggaagtccag (13) | Paul et al., Na-ture Biotech, 20: 505-8 (2002). |

**Table 2: shRNA sequences known from Brummelkamp et al., Nature, 424:797-801 (2003)**

| Target Gene | shRNA Sequence (SEQ ID NO) |
|---|---|
| UBIQUITIN CARBOXYL-TERMINAL HYDROLASE 12 | GAGATTGGTCCAGAACAGTTTCAAGAGAACTGTTCTGGACCAATCTC (14) GCCCTTCCGATCATGGTAGTTCAAGAGACTACCATGATCGGAAGGGC (15) TCTTTAGAATTCTTAAGTATTCAAGAGATACTTAAGAATTCTAAAGA (16) CATTAGCTATATCAACATGTTCAAGAGACATGTTGATATAGCTAATG (17) |
| UBIQUITIN CARBOXYL-TERMINAL HYDROLASE 11 | ACCACAAACGGCGGAACGATTCAAGAGATCGTTCCGCCGTTTGTGGT (18) GAGGGTCTTGGAGGTCTTCTTCAAGAGAGAAGACCTCCAAGACCCTC (19) GTCCATGCCCAGCCGTACATTCAAGAGATGTACGGCTGGGCATGGAC (20) GCTGGACACCCTCGTGGAGTTCAAGAGACTCCACGAGGGTGTCCAGC (21) |
| UBIQUITIN CARBOXYL-TERMINAL HYDROLASE 10 | GAATATCAGAGAATTGAGTTTCAAGAGAACTCAATTCTCTGATATTC (22) TGGACTTCATGAGGAAATGTTCAAGAGACATTTCCTCATGAAGTCCA (23) TATTGAATATCCTGTGGACTTCAAGAGAGTCCACAGGATATTCAATA (24) TTGTACTGAGAGAAACTGCTTCAAGAGAGCAGTTTCTCTCAGTACAA (25) |
| HAUSP | GATCAATGATAGGTTTGAATTCAAGAGATTCAAACCTATCATTGATC (26) GGAGTTTGAGAAGTTTAAATTCAAGAGATTTAAACTTCTCAAACTCC (27) GAACTCCTCGCTTGCTGAGTTCAAGAGACTCAGCAAGCGAGGAGTTC (28) CCGAATTTAACAGAGAGAATTCAAGAGATTCTCTCTGTTAAATTCGG (29) |
| UBIQUITIN CARBOXYL-TERMINAL HYDROLASE 8 | GACAGCAGAAGAATGCAGATTCAAGAGATCTGCATTCTTCTGCTGTC (30) ATAAAGCTCAACGAGAACCTTCAAGAGAGGTTCTCGTTGAGCTTTAT (31) GGTGAAGTGGCAGAAGAATTTCAAGAGAATTCTTCTGCCACTTCACC (32) GTATTGCAGTAATCATCACTTCAAGAGAGTGATGATTACTGCAATAC (33) |
| FLJ10785 | GATATGGGGTTCCATGTCATTCAAGAGATGACATGGAACCCCATATC (34) GGAGACATGGTTCTTAGTGTTCAAGAGACACTAAGAACCATGTCTCC (35) AGCACCAAGTTCGTCTCAGTTCAAGAGACTGAGACGAACTTGGTGCT (36) GATGCAACACTGAAAGAACTTCAAGAGAGTTCTTTCAGTGTTGCATC (37) |
| KIAA0710 | GTCAATGGCAGTGATGATATTCAAGAGATATCATCACTGCCATTGAC (38) CCTGCTAGCTGCCTGTGGCTTCAAGAGAGCCACAGGCAGCTAGCAGG (39) CCACCTTTGCCAGAAGGAGTTCAAGAGACTCCTTCTGGCAAAGGTGG (40) CCCTATTGAGGCAAGTGTCTTCAAGAGAGACACTTGCCTCAATAGGG (41) |
| FLJ12552/ FLJ14256 | GAAGGAAAACTTGCTGACGTTCAAGAGACGTCAGCAAGTTTTCCTTC (42) CTCACCTGGGTCCATGAGATTCAAGAGATCTCATGGACCCAGGTGAG (43) GCTGTCTTACCGTGTGGTCTTCAAGAGAGACCACACGGTAAGACAGC (44) CCTGGACCGCATGTATGACTTCAAGAGAGTCATACATGCGGTCCAGG (45) |
| KIAA1203 | GTCAATGGCAGTGATGATATTCAAGAGATATCATCACTGCCATTGAC (46) CCTGCTAGCTGCCTGTGGCTTCAAGAGAGCCACAGGCAGCTAGCAGG (47) CCACCTTTGCCAGAAGGAGTTCAAGAGACTCCTTCTGGCAAAGGTGG (48) CCCTATTGAGGCAAGTGTCTTCAAGAGAGACACTTGCCTCAATAGGG (49) |
| FLJ23277 | GGAAATCCGAATTGCTTGGTTCAAGAGACCAAGCAATTCGGATTTCC (50) CACATTTCTTCAAGTGTGGTTCAAGAGACCACACTTGAAGAAATGTG (51) CAGCAGGATGCTCAAGAATTTCAAGAGAATTCTTGAGCATCCTGCTG (52) GCTGAATACCTACATTGGCTTCAAGAGAGCCAATGTAGGTATTCAGC (53) |
| FLJ14914 (similar to UBP4) | GGGCTTGTGCCTGGCCTTGTTCAAGAGACAAGGCCAGGCACAAGCCC (54) GCCTTGTCCTGCCAAGAAGTTCAAGAGACTTCTTGGCAGGACAAGGC (55) GATTGAAGCCAAGGGAACGTTCAAGAGACGTTCCCTTGGCTTCAATC (56) TGGCGCCTGCTCCCCATCTTTCAAGAGAAGATGGGGAGCAGGCGCCA (57) |
| UBIQUITIN CAR- BOXYL-TERMINAL HYDROLASE ISOZYME L5 | GAACCAGCAGGCTCTGTGGTTCAAGAGACCACAGAGCCTGCTGGTTC (58) GGAAGCATAATTATCTGCCTTCAAGAGAGGCAGATAATTATGCTTCC (59) AGAAGAAGATGCTTTTCACTTCAAGAGAGTGAAAAGCATCTTCTTCT (60) CTTGCAGAGGAGGAACCCATTCAAGAGATGGGTTCCTCCTCTGCAAG (61) |
| UBIQUITIN CAR- BOXYL-TERMINAL HYDROLASE ISOZYME L3 | GCAAACAATCAGCAATGCCTTCAAGAGAGGCATTGCTGATTGTTTGC (62) TTGGACTGATTCATGCTATTTCAAGAGAATAGCATGAATCAGTCCAA (63) CTGGCAATTCGTTGATGTATTCAAGAGATACATCAACGAATTGCCAG (64) TTAGATGGGCGGAAGCCATTTCAAGAGAATGGCTTCCGCCCATCTAA (65) |
| UBIQUITIN CAR-BOXYL-TERMINAL HYDROLASE ISOZYME L1 | GAGGAGTCTCTGGGCTCGGTTCAAGAGACCGAGCCCAGAGACTCCTC (66) GAGCTGAAGGGACAAGAAGTTCAAGAGACTTCTTGTCCCTTCAGCTC (67) TGTCGGGTAGATGACAAGGTTCAAGAGACCTTGTCATCTACCCGACA (68) CACAGCTGTTCTTCTGTTCTTCAAGAGAGAACAGAAGAACAGCTGTG (69) |
| KIAA1891 / FLJ25263 | GTGGAAGCCTTTACAGATCTTCAAGAGAGATCTGTAAAGGCTTCCAC (70) CAACAGCTGCCTTCATCTGTTCAAGAGACAGATGAAGGCAGCTGTTG (71) CCATAGGCAGTCCTCCTAATTCAAGAGATTAGGAGGACTGCCTATGG (72) TGTATCACTGCCACTGGTTTCAAGAGAAACCAGTGGCAGTGATACA (73) |
| FLJ14528 (similar to UBP8) | r CATGTTGGGCAGCTGCAGCTTCAAGAGAGCTGCAGCTGCCCAACATG (74) CACAACTGGAGACCTGAAGTTCAAGAGACTTCAGGTCTCCAGTTGTG (75) GTATGCCTCCAAGAAAGAGTTCAAGAGACTCTTTCTTGGAGGCATAC (76) CTTCACAGTACATTTCTCTTTCAAGAGAAGAGAAATGTACTGTGAAG (77) |
| U4/U6 TR1 SNRNP 65 kDa protein | PGTACTTTCAAGGCCGGGGTTTCAAGAGAACCCCGGCCTTGAAAGTAC (78) CTTGGACAAGCAAGCCAAATTCAAGAGATTTGGCTTGCTTGTCCAAG (79) GACTATTGTGACTGATGTTTTCAAGAGAAACATCAGTCACAATAGTC (80) GGAGAACTTTCTGAAGCGCTTCAAGAGAGCGCTTCAGAAAGTTCTCC (81) |
| XM_089437 | GACGAGAGAAACCTTCACCTTCAAGAGAGGTGAAGGTTTCTCTCGTC (82) ACATTATTCTACATTCTTTTCAAGAGAAAAGAATGTAGAATAATGT (83) AGATTCGCAAATGGATGTATTCAAGAGATACATCCATTTGCGAATCT (84) CATTCCCACCATGAGTCTGTTCAAGAGACAGACTCATGGTGGGAATG (85) |
| KIAA1453 | GATCGCCCGACACTTCCGCTTCAAGAGAGCGGAAGTGTCGGGCGATC (86) CCAGCAGGCCTACGTGCTGTTCAAGAGACAGCACGTAGGCCTGCTGG (87) GCCAGCTCCTCCACAGCACTTCAAGAGAGTGCTGTGGAGGAGCTGGC (88) CGCCGCCAAGTGGAGCAGATTCAAGAGATCTGCTCCACTTGGCGGCG (89) |
| FLJ12697 | GAAGATGCCCATGAATTCCTTCAAGAGAGGAATTCATGGGCATCTTC (90) CAAACAGGCTGCGCCAGGCTTCAAGAGAGCCTGGCGCAGCCTGTTTG (91) ACGGCCTAGCGCCTGATGGTTCAAGAGACCATCAGGCGCTAGGCCGT (92) |
| | CTGTAACCTCTCTGATCGGTTCAAGAGACCGATCAGAGAGGTTACAG (93) |
| UBIQUITIN SPECIFIC PROTEASE 18 (USP18) | TCTGTCAGTCCATCCTGGCTTCAAGAGAGCCAGGATGGACTGACAGA (94) TGAAGCGAGAGTCTTGTGATTCAAGAGATCACAAGACTCTCGCTTCA (95) GATGGAGTGCTAATGGAAATTCAAGAGATTTCCATTAGCACTCCATC (96) CCTTCAGAGATTGACACGCTTCAAGAGAGCGTGTCAATCTCTGAAGG (97) |
| UBIQUITIN CARBOXYL-TERMINAL HYDROLASE 20 | CCTGACCACGTTCCGACTGTTCAAGAGACAGTCGGAACGTGGTCAGG (98) GAGTTCCTTCGCTGCCTGATTCAAGAGATCAGGCAGCGAAGGAACTC (99) GACTGCCTTGCTGCCTTCTTTCAAGAGAAGAAGGCAGCAAGGCAGTC (100) CGCCGAGGGCTACGTACTCTTCAAGAGAGAGTACGTAGCCCTCGGCG (101) |
| UBIQUITIN CARBOXYL-TERMINAL HYDROLASE 24 | GGCGAGAAGAAAGGACTGTTTCAAGAGAACAGTCCTTTCTTCTCGCC (102) GGACGAGAATTGATAAAGATTCAAGAGATCTTTATCAATTCTCGTCC (103) GCACGAGAATTTGGGAATCTTCAAGAGAGATTCCCAAATTCTCGTGC (104) CTACTTCATGAAATATTGGTTCAAGAGACCAATATTTCATGAAGTAG (105) |
| KIAA1594 | GATAACAGCTTCTTGTCTATTCAAGAGATAGACAAGAAGCTGTTATC (106) GAGAATAGGACATCAGGGCTTCAAGAGAGCCCTGATGTCCTATTCTC (107) CTTGGAAGACTGAACCTGTTTCAAGAGAACAGGTTCAGTCTTCCAAG (108) CAACTCCTTTGTGGATGCATTCAAGAGATGCATCCACAAAGGAGTTG (109) |
| KIAA1350 | GATGTTGTCTCCAAATGCATTCAAGAGATGCATTTGGAGACAACATC (110) CGTGGGGACTGTACCTCCCTTCAAGAGAGGGAGGTACAGTCCCCACG (111) GTACAGCTTCAGAACCAAGTTCAAGAGACTTGGTTCTGAAGCTGTAC (112) |
| UBIQUITIN CARBOXYL- TERMINAL HYDROLASE 25 | GATGATCTTCAGAGAGCAATTCAAGAGATTGCTCTCTGAAGATCATC (113) GGAACATCGGAATTTGCCTTTCAAGAGAAGGCAAATTCCGATGTTCC (114) GAGCTAGTGAGGGACTCTTTTCAAGAGAAAGAGTCCCTCACTAGCTC (115) GCAGGGTTCTTTAAGGCAATTCAAGAGATTGCCTTAAAGAACCCTGC (116) |
| UBIQUITIN CARBOXYL-TERMINAL HYDROLASE 16 | TCGATGATTCCTCTGAAACTTCAAGAGAGTTTCAGAGGAATCATCGA (117) GATAATGGAAATATTGAACTTCAAGAGAGTTCAATATTTCCATTATC (118) GTTCTTCATTTAAATGATATTCAAGAGATATCATTTAAATGAAGAAC (119) GTTAACAAACACATAAAGTTTCAAGAGAACTTTATGTGTTTGTTAAC (120) |
| USP9X | GTTAGAGAAGATTCTTCGTTTCAAGAGAACGAAGAATCTTCTCTAAC (121) GTTGATTGGACAATTAAACTTCAAGAGAGTTTAATTGTCCAATCAAC (122) GGTTGATACCGTAAAGCGCTTCAAGAGAGCGCTTTACGGTATCAACC (123) GCAATGAAACGTCCAATGGTTCAAGAGACCATTGGACGTTTCATTGC (124) |
| USP9Y | AGCTAGAGAAAATTCTTCGTTCAAGAGACGAAGAATTTTCTCTAGCT (125) GATCCTATATGATGGATGATTCAAGAGATCATCCATCATATAGGATC (126) GTTCTTCTTGTCAGTGAAATTCAAGAGATTTCACTGACAAGAAGAAC (127) CTTGAGCTTGAGTGACCACTTCAAGAGAGTGGTCACTCAAGCTCAAG (128) |
| UBIQUITIN CARBOXYL-TERMINAL HYDROLASE 5 | GACCGGCCAGCGAGTCTACTTCAAGAGAGTAGACTCGCTGGCCGGTC (129) GGACCTGGGCTACATCTACTTCAAGAGAGTAGATGTAGCCCAGGTCC (130) CTCTGTGGTCCAGGTGCTCTTCAAGAGAGAGCACCTGGACCACAGAG (131) GACCACACGATTTGCCTCATTCAAGAGATGAGGCAAATCGTGTGGTC (132) |
| UBIQUITIN CARBOXYL- | TGGCTTGTTTATTGAAGGATTCAAGAGATCCTTCAATAAACAAGCCA (133) GTGAATTTGGGGAAGATAATTCAAGAGATTATCTTCCCCAAATTCAC (134) |
| TERMINAL HYDROLASE 26 | CGCTATAGCTTGAATGAGTTTCAAGAGAACTCATTCAAGCTATAGCG (135) GATATCCTGGCTCCACACATTCAAGAGATGTGTGGAGCCAGGATATC (136) |
| KIAA1097 | GAGCCAGTCGGATGTAGATTTCAAGAGAATCTACATCCGACTGGCTC (137) GTAAATTCTGAAGGCGAATTTCAAGAGAATTCGCCTTCAGAATTTAC (138) GCCCTCCTAAATCAGGCAATTCAAGAGATTGCCTGATTTAGGAGGGC (139) GTTGAGAAATGGAGTGAAGTTCAAGAGACTTCACTCCATTTCTCAAC (140) |
| UBIQUITIN SPECIFIC PROTEASE 22 (USP22) | GCTTGGAAAATGCAAGGCGTTCAAGAGACGCCTTGCATTTTCCAAGC (141) CTGCATCATAGACCAGATCTTCAAGAGAGATCTGGTCTATGATGCAG (142) GATCACCACGTATGTGTCCTTCAAGAGAGGACACATACGTGGTGATC (143) TGACAACAAGTATTCCCTGTTCAAGAGACAGGGAATACTTGTTGTCA (144) |
| UBIQUITIN-SPECIFIC PROCESSING PROTEASE 29 | GAAATATAAGACAGATTCCTTCAAGAGAGGAATCTGTCTTATATTTC (145) CCCATCAAGTTTAGAGGATTTCAAGAGAATCCTCTAAACTTGATGGG (146) GGTGTCCCATGGGAATATATTCAAGAGATATATTCCCATGGGACACC (147) GAATGCCGACCTACAAAGATTCAAGAGATCTTTGTAGGTCGGCATTC (148) |
| CYLD | CAGTTATATTCTGTGATGTTTCAAGAGAACATCACAGAATATAACTG (149) GAGGTGTTGGGGACAAAGGTTCAAGAGACCTTTGTCCCCAACACCTC (150) GTGGGCTCATTGGCTGAAGTTCAAGAGACTTCAGCCAATGAGCCCAC (151) GAGCTACTGAGGACAGAAATTCAAGAGATTTCTGTCCTCAGTAGCTC (152) |
| UBIQUITIN CARBOXYL-TERMINAL HYDROLASE 2 | TCAGCAGGATGCTCAGGAGTTCAAGAGACTCCTGAGCATCCTGCTGA (153) GAAGTTCTCCATCCAGAGGTTCAAGAGACCTCTGGATGGAGAACTTC (154) GCCGGTCCCCACCAGCAGCTTCAAGAGAGCTGCTGGTGGGGACCGGC (155) CACTCGGGAGTTGAGAGATTTCAAGAGAATCTCTCAACTCCCGAGTG (156) |
| UBIQUITIN SPECIFIC PROTEASE 3 (USP3) | GCCCTTGGGTCTGTTTGACTTCAAGAGAGTCAAACAGACCCAAGGGC (157) CTCAACACTAAACAGCAAGTTCAAGAGACTTGCTGTTTAGTGTTGAG (158) GATTTCATTGGACAGCATATTCAAGAGATATGCTGTCCAATGAAATC (159) CATGGGGCACCAACTAATTTTCAAGAGAAATTAGTTGGTGCCCCATG (160) |
| UBIQUITIN CARBOXYL-TERMINAL HYDROLASE 23 | GGTGTCTCTGCGGGATTGTTTCAAGAGAACAATCCCGCAGAGACACC (161) AGTTCAGTAGGTGTAGACTTTCAAGAGAAGTCTACACCTACTGAACT (162) GAGTTCCTGAAGCTCCTCATTCAAGAGATGAGGAGCTTCAGGAACTC (163) GGATTTGCTGGGGGCAAGGTTCAAGAGACCTTGCCCCCAGCAAATCC (164) |
| UBP-32.7 | CTCAGAAAGCCAACATTCATTCAAGAGATGAATGTTGGCTTTCTGAG (165) CGCATTGTAATAAGAAGGTTTCAAGAGAACCTTCTTATTACAATGCG (166) GGGAGGAAAATGCAGAAATTTCAAGAGAATTTCTGCATTTTCCTCCC (167) TTACAAATTTAGGAAATACTTCAAGAGAGTATTTCCTAAATTTGTAA (168) |
| HOMO SAPIENS UBIQUITIN SPE-CIFIC PROTEASE 13 (ISOPEP-TIDASE T-3) | GTTATGAATTGATATGCAGTTCAAGAGACTGCATATCAATTCATAAC (169) GTGATAACACAACTAATGGTTCAAGAGACCATTAGTTGTGTTATCAC (170) GTAGAGGAGAGTTCTGAAATTCAAGAGATTTCAGAACTCTCCTCTAC (171) GCCTCTAATCCTGATAAGGTTCAAGAGACCTTATCAGGATTAGAGGC (172) |
| UBIQUITIN CARBOXYL-TERMINAL HYDROLASE 28 | GATGATCTTCAGGCTGCCATTCAAGAGATGGCAGCCTGAAGATCATC (173) GTATGGACAAGAGCGTTGGTTCAAGAGACCAACGCTCTTGTCCATAC (174) CGAACCCTTCTGGAACAGTTTCAAGAGAACTGTTCCAGAAGGGTTCG (175) GTGGCATGAAGATTATAGTTTCAAGAGAACTATAATCTTCATGCCAC (176) |
| UBIQUITIN CARBOXYL-TERMINAL HYDROLASE 14 | GGTGAACAAGGACAGTATCTTCAAGAGAGATACTGTCCTTGTTCACC (177) GCAATAGAGGATGATTCTGTTCAAGAGACAGAATCATCCTCTATTGC (178) TCTGTGAATGCCAAAGTTCTTCAAGAGAGAACTTTGGCATTCACAGA (179) CACACCAGGGAAGGTCTAGTTCAAGAGACTAGACCTTCCCTGGTGTG (180) |
| DUB1 | GCAGGAAGATGCCCATGAATTCAAGAGATTCATGGGCATCTTCCTGC (181) GAATGTGCAATATCCTGAGTTCAAGAGACTCAGGATATTGCACATTC (182) TGGATGATGCCAAGGTCACTTCAAGAGAGTGACCTTGGCATCATCCA (183) GCTCCGTGCTAAACCTCTCTTCAAGAGAGAGAGGTTTAGCACGGAGC (184) |
| MOUSE USP27 HOMOLOG | GCCTCCACCTCAACAGAGGTTCAAGAGACCTCTGTTGAGGTGGAGGC (185) CTGCATCATAGACCAAATCTTCAAGAGAGATTTGGTCTATGATGCAG (186) GATCACTACATACATTTCCTTCAAGAGAGGAAATGTATGTAGTGATC (187) GTAAAGAGAGCAGAATGAATTCAAGAGATTCATTCTGCTCTCTTTAC (188) |
| UBIQUITIN CARBOXYL-TERMINAL HYDROLASE 4 | CGCGGGGCGCAGTGGTATCTTCAAGAGAGATACCACTGCGCCCCGCG (189) CAGAAGGCAGTGGGGAAGATTCAAGAGATCTTCCCCACTGCCTTCTG (190) GCCTGGGAGAATCACAGGTTTCAAGAGAACCTGTGATTCTCCCAGGC (191) ACCAGACAAGGAAATACCCTTCAAGAGAGGGTATTTCCTTGTCTGGT (192) |
| TRE-2 | CACATCCACCACATCGACCTTCAAGAGAGGTCGATGTGGTGGATGTG (193) GTCACAACCCAAGACCATGTTCAAGAGACATGGTCTTGGGTTGTGAC (194) CTCAACAGGACAAATCCCATTCAAGAGATGGGATTTGTCCTGTTGAG (195) TAGATCAATTATTGTGGATTTCAAGAGAATCCACAATAATTGATCTA (196) |
| UBIQUITIN CAR-BOXYL-TERMINAL HYDROLASE 15 (UNPH-2). | GGAACACCTTATTGATGAATTCAAGAGATTCATCAATAAGGTGTTCC (197) L CTTTAACAGAAATTGTCTCTTCAAGAGAGAGACAATTTCTGTTAAAG (198) CCTATGCAGTACAAAGTGGTTCAAGAGACCACTTTGTACTGCATAGG (199) GATCTTTTCTTGCTTTGGATTCAAGAGATCCAAAGCAAGAAAAGATC (200) |
| KIAA1372 | CAGCATCCTTCAGGCCTTATTCAAGAGATAAGGCCTGAAGGATGCTG (201) GATAGTGACTCGGATCTGCTTCAAGAGAGCAGATCCGAGTCACTATC (202) GACATCACAGCCCGGGAGTTCAAGAGAACTCCCGGGCTGTGATGTC (203) GGACACAGCCTATGTGCTGTTCAAGAGACAGCACATAGGCTGTGTCC (204) |
| BRCA1 ASSOCIATED PROTEIN-1 | GTGGAGGAGATCTACGACCTTCAAGAGAGGTCGTAGATCTCCTCCAC (205) CTCTTGTGCAACTCATGCCTTCAAGAGAGGCATGAGTTGCACAAGAG (206) ACAGGGCCCCTGCAGCCTCTTCAAGAGAGAGGCTGCAGGGGCCCTGT (207) GAAGACCTGGCGGCAGGTGTTCAAGAGACACCTGCCGCCAGGTCTTC (208) |

Suitable siRNA sequences for the knockdown of a given target gene are well known in the art ( e.g. the particular siRNA sequences mentioned in Lee N.S. et al., J. Nat. Biotechnol. 20(5):500-5 (2002) gcctgtgcctcttcagctacc (SEQ ID NO:209) and gcggagacagcgacgaagagc (SEQ ID NO:210) and in Du, Q. et al., Nucl. Acids Res. 21; 33(5):1671-7 (2005) cttattggagagagcacga (SEQ ID NO:211)) or can readily be determined by the skilled artisan.

It is furthermore preferred in the regulator construct of the lentiviral vector of aspect (1) of the invention that the repressor gene is under control of an ubiquitous promoter preferably selected from polymerase (Pol) I, II and III dependent promoters, preferably is a Pol II dependent promoter, more preferably is a CMV promoter, a CAGGS promoter or a Ubiquitin promoter.

Furthermore it is preferred in the regulator construct that the repressor gene is a codon-optimized tet repressor, a codon-optimized Gal4 repressor, a codon-optimized lac repressor or a variant thereof, preferably is a codon-optimized tet repressor having the sequence of bp 4206 to 4826 of SEQ ID NO:1.

Still further preferred is that the marker is a fluorescence marker, preferably selected from green fluorescent protein (GFP), DsRed, AsRed, HcRed, Tomatoe, Cherry, Katushka, and variants thereof, most preferably is eGFP having the sequence shown of bp 4896 to 5609 of SEQ ID NO:1. For further selectable markers it is referred to U.S. Patents Nos. 5,487,932 and 5,464,763 which are hereby incorporated in their entirety.

In a preferred embodiment of aspect (2) of the invention the lentiviral vector has the sequence shown in SEQ ID NO:1

In the biological entity of embodiment (3) of the invention it is preferred that the responder construct and/or the regulator construct is (are) stably integrated into the genome of the biological entity, preferably at actively transcribed regions, by viral integration or the like, preferably by viral transduction with a lentiviral vector of aspects (1) or (2) above. It is moreover preferred that the responder construct and the regulator construct are integrated at the same locus in the genome of the biological entity, preferably said biological entity carries the sequence of bp 839 to 6973 of SEQ ID NO:1.

Further it is preferred that the vertebrate is a non-human vertebrate, preferably is a rodent, such as mouse or rat, pig, cattle, sheep, bird or a fish, most preferably the vertebrate is a rat or mouse.

Moreover it is preferred that the cells of the vertebrate carrying a responder construct and a regulator construct as defined in aspects (1) and (2) above are mammalian cells including human cells, preferably are primary cells stem cells and zygotes (but not including human stem cells and zygotes).

It is particularly preferred that the biological entity is a rat, rat cell or ratmouse tissue, and the responder and regulator construct are as defined in aspects (1) and (2) above.

The present invention is hereinafter more specifically explained with reference to the specific experimental results. This explanation is, however not to be construed as a limitation of the invention.

For the inducible expression of shRNAs a RNA polymerase III-dependent H1 promoter, containing operator sequences (tetO) of the *E. coli* tetracycline resistance operon, was incorporated. Binding of the tetracycline resistance operon repressor (tetR) to tetO blocks transcription, whereas de-repression is achievable by adding the inductor doxycycline (dox), causing release of tetR. The tetR coding region is under control of the RNA polymerase II-dependent Ubiquitin promoter downstream of the H1-tetO-shRNA cassette. Contrary to other published systems we achieve a very tight regulation of the shRNA due to the codon optimization of the tetR, which allows very tight repression and de-repression of the shRNA. Additionally we incorporated a marker gene (GFP) into the lentiviral construct, which is also under control of the ubiquitin promoter and linked downstream of the tetR. In contrast to earlier vector constructs the linkage of the tetR and GFP is not via a second ribosomal binding site (IRES) or a fusion protein, but through placing a viral peptide sequence (T2A) between both proteins. Initially this leads to the generation of a bicistronic mRNA, containing tetR and GFP. Due to the viral sequence however, the ribosomes do not form a peptide bond between the C-terminus of the tetR and the N-terminus of the GFP, resulting in two independent proteins. This approach has two major advantages: (i) the GFP expression is very high, reliable and allows for the tracking of shRNA positive cells and (ii) the formation of two independent proteins avoids a loss of the tetR function by generating a tetR-GFP fusion protein. Finally the H1-tetO-shRNA cassette can be easily exchanged, through the usage of a single restriction site.

The configuration of the vector was tested *in vitro* in a rat T-cell Hybridoma cell line for the inducible knock down of the surface receptor CD8 alpha. The down regulation of this protein after adding doxycycline in GFP positive cells could be shown. By withdrawing doxycycline out of the cell cultures the knock down leading to the re-expression of CD8 alpha on the cell surface could be reversed.

The generation of transgenic animals with a single vector allowing the inducible expression of shRNAs. The lentiviral vector described in (1) and (2) above can also be used for the generation of transgenic animals by transducing fertilized oocytes. We employed this technique for the generation of transgenic rats expressing an inducible shRNA targeting the rat insulin receptor (IRtg). Due to the GFP marker in the lentiviral vector we could easily identify founder lines, having a functional lentiviral sequence incorporated into their genome. As described in (1) we used the first time a viral peptide sequence for a simultaneous expression of the tetR and GFP. Especially in transgenic animals this is crucial, because the use of an IRES sequence often goes along with a non-reliable expression of the linked gene in several cell types (Creancier, L., Morello, D., Mercier, P., and Prats, A. C. (2000) J Cell Biol. 150(1), 275-281). Due to an equal expression pattern of the messenger RNA in all cell types, we get the same degree of tetR protein, necessary for tight control of the shRNA induction. By adding very low doses of doxycycline into the drinking water of IRtg rats we observed a very fast increase in blood glucose concentrations, indicating a defective sugar metabolism in those animals. Further analysis showed that this increase was due to a strong down regulation of the Insulin receptor in response to a doxycycline-induced expression of the shRNA. Most importantly blood sugar levels declined to the basal levels when removing doxycycline from the drinking water of those rats. This model shows that the lentiviral vector described in (1) can be efficiently used to generate transgenic animals with an inducible shRNA cassette, which is tightly regulated and revertable.

Taken together this system is the first lentiviral based shRNA approach, combining all necessary "items" in a single configuration for reliable induction and repression of the shRNA for *in vitro* and *in vivo* applications.

The advantages are
(i) the generation of one transgenic line, thereby preventing complicated crossing schemes,
(ii) the tight regulation makes the usage of very high dox doses unnecessary, which will exclude possible side effects through the drug,
(iii) the strong expression of GFP allows easy identification of transgenic lines avoiding complicated PCRs and/ or Southern Blot technologies and
(iv) the ubiquitous usage of lentiviral particles in tumor cell lines, primary cells, transgenic models (rats, mice, birds, etc.) and even primary human cells as a possible tool for gene therapy.

For further selectable markers it is referred to U.S. Patents Nos. 5,487,932 and 5,464,763, which are hereby incorporated in their entirety.

The invention also provides a method for preparing a vertebrate, i.e. a transgenic multi-cell organism carrying the shRNA vector construct of aspects (1) and (2) above, notably at a actively expressed loci. This includes a method for preparing a non-human mammal comprising modifying starting ES cells according to steps (a) to (c). The ES cells may subsequently processed according one or more of the following steps: (d) the ES cells obtained in steps (b) or (c) are injected into blastocysts; and/or (e) transgenic non-human animals carrying the shRNA vector construct are generated (viz. by well known breeding procedures).

The invention is further described in the following Examples that are, however, not to be construed as a limitation of the invention.

### Examples

### Materials and Methods

Inducible lentiviral shRNA construct: To generate an inducible lentiviral vector construct, the FUGW (Lois, C. et al., Science 295(5556), 868-872(2002)) was used as a backbone and the PCR amplified H1-tetO-shRNA cassette (Seibler, J. et al., Nucleic Acids Res. 35(7), e54(2007)) was incorporated via PacI restriction upstream of the Ubiquitin promoter. The codon optimized Tet-Repressor sequence was amplified from pRMCE-tetO-htetRinv-IR5 (Seibler, J. et al., Nucleic Acids Res. 35(7), e54(2007)) and fused with the T2A (Szymczak, A. L., and Vignali, D. A., Expert Opin. Biol. Ther. 5(5), 627-638 (2005)) peptide onto eGFP. This fusion product was cloned downstream of the Ubiquitin promoter via a BamH1 and MfeI/ EcoRI digestion. For the Insulin Receptor knock-down we amplified the H1-tet-shIR5 cassette from pRMCE-tetO-htetRinv-IR5 (Seibler, J. et al., Nucleic Acids Res. 35(7), e54 (2007)). The shCD8 alpha sequence used 5'-CGGAGCAAGCTGAACGATATATttcaagagaGTATATCGTTCAGCTTGCTCCG-3' (SEQ ID NO:2) was cloned into the H1-tet-flex plasmid (Seibler, J. et al., Nucleic Acids Res. 35(7), e54 (2007)) via BbsI and XhoI. Afterwards the H1-tet-shCD8 alpha cassette was PCR amplified and cloned into the PacI site of the inducible vector construct.

Virus production and transduction of cells: For the production of viral particles, we transfected HEK293T cells with 10 µg lentiviral vector plus helper plasmids (pMDL-RRE 5 µg, pRSV-REV 2.5 µg, pVSV-g 3 µg) using CaPO₄ precipitation (Pfeifer, A. et al., Proc. Natl. Acad. Sci. U S A 97(22), 12227-12232 (2000); Dull, T. et al., J. Virol. 72(11), 8463-8471 (1998); Naldini, L. et al., Science 272(5259), 263-267 (1996)). 48 h after transfection viral supernatants were collected and filtered through a 0.45 µm Filter. For concentrating the virus 40 ml of viral supernatants were centrifuged in a swinging bucket (Surespin) for 90 min at 25000 rpm. Afterwards the virus pellet was resuspended in 30 µl of PBS/ 0.1% BSA. Viral titers reached were 1 - 2x10⁸ TU/ml.

Cell Culture: HEK293T cells were maintained in Dulbecco's modified Eagle's medium (DMEM) 10%FCS, 1:100 Penicillin / Streptomycin. The T-cell Hybridoma cell line was maintained in RPMI, 10% FCS, 1:1000 β-Me and 1:100 Penicillin / Streptomycin.

Transduction of cell lines: 1x10⁵ Hybridoma T-cells were transduced by spinning with 5 ml virus supernatant and 10 µg/ml Polybrene for 3 h at 2200 rpm. Three days later the transduction efficiency was measured on the basis of the eGFP expression at the FACS.

Doxycycline treatment: Hybridoma cells were treated with 1 µg/ml doxycycline (Sigma D-9891). Medium containing doxycycline was prepared and changed every second day.

CD8 alpha analysis: 4x10⁵ Hybridoma cells were harvested and resuspended in 100 µl FACS-buffer (PBS, 0.1%BSA, 0.1% sodium azide) and incubated with PerCP labelled anti-CD8 alpha antibody (OX-8-PerCP; BD) for 20 min. Cells were washed in FACS buffer and subsequently analyzed by flow cytometry.

Generation of transgenic rats: Transgenesis was performed according to standard protocols (van den Brandt, J. et al., Genesis 39(2), 94-99 (2004)). The lentiviral concentrate (10-100 pl) was injected into the perivitelline space of single-cell rat embryos obtained from superovulated Lewis (Lew/Crl) females mated on the previous evening with Lew/Crl males (Charles River, Sulzfeld, Germany). The injected zygotes were cultured overnight and the next morning two-cell stage embryos were transferred into the oviduct of pseudopregnant CrL/CD females. All experiments were conducted in accordance with accepted standards of humane animal care. For doxycycline treatment Solutions containing 2 mg/ml doxycycline (Sigma D-9891), 10% sucrose or 20 µg/ml doxycycline, 1% sucrose or 2 µg/ml doxycycline, 0.1% sucrose in drinking water were prepared every second day and kept dark.

Protein isolation: Cells were lysed in protein extraction buffer containing 1% NP-40, 0.1% SDS, 0.5% sodium deoxycholate, 97.5% PBS, and freshly added protease inhibitor cocktail (Roche). Protein concentration was calculated using the Bio-Rad protein reagent (BioRad).

Western blot: Proteins were fractionated on a 10% SDS-PAGE gel and semi-dry blotted for 120 min at 70 mA. Primary antibodies: INSR (Rβ (C-19) #sc-711, Santa Cruz Biotechnology) was diluted 1:200, tet02 (Mobitec, #tet02) 1:500 and ERK (C-14; sc-154) in PBS/0.1% Tween containing 5% milk powder. Goat anti-rabbit IgG-HRP and goat anti-mouse IgG-HRP (Dianova) were used as secondary antibodies. ECL reagent for detection of protein was used according to manufacturer's instructions (Pierce).

RNA isolation: Tissue was homogenized and subsequently RNA was isolated in Trizol reagent (Invitrogen) according to manufacturer's protocol.

Northern blot: 15 µg RNA was fractionated on a 15% denaturating polyacrylamide gel and blotted on a nylon membrane at 35 mA for 90 min. RNA was cross-linked to the membrane using UV-light and incubated at 80°C for 30 min. The membrane was subsequently incubated for 2 h in 10 ml pre-hybridization solution (Clontech) and labeled using radioactive, oligo probes against antisense strand of shRNA-IR5 (gaccagacccgaagatttct; SEQ ID NO:3) and against 5srRNA (tcctgcaattcacattaattctcgcagctagc; SEQ ID NO:4). A 10 U T4-Polynucleotide-kinase (Fermentas) and 10 µCi - (³²P)-ATP (10 U µCi/µl) were used for probe labeling.

### Example 1: Design of a shRNA inducible lentiviral vector

To generate a single lentiviral vector system for a inducible and reversible gene knockdown, the codon optimized Tet-Repressor was inserted 5' of the eGFP gene under the control of the ubiquitin promoter. The linkage of the TetR and eGFP, was achieved by placing the viral T2A peptide between the two coding sequences, allowing strong and independent expression of both gene products (Fig1a). Initial experiments showed the functionality of the linkage strategy, because both eGFP and the right sized tetR could be detected by flow cytometric analysis and western blotting respectively (Fig. 1b). Upstream of the ubiquitin promoter a shRNA cassette was inserted that contained the RNA-polymerase-III promoter H1 with inserted tetO sequences 3' of the TATA box (Fig. 1a). To test the inducibility and reversibility of the system, a shRNA targeting the surface protein CD8 alpha was designed (FH1tshCD8aUTG,). Cells transduced with FH1tshCD8aUTG showed the same CD8 alpha expression on their cell surface than non-transduced cells in the absence of doxycycline (dox) (Fig. 2a). Addition of dox for seven days led to a down regulation of CD8 alpha on transduced cells (Fig2b). Another 10 days later in medium alone, transduced cells reached nearly the same CD8 alpha expression than wild-type cells (Fig. 2c).

### Example 2: Inducible knockdown of the Insulin receptor in rats

For testing the inducible single vector system *in vivo*, the insulin receptor was chosen as the target gene, because in a mouse model a functional shRNA was already described which shared 100% homology with the rat sequence. For that purpose transgenic rats were generated with lentiviruses for the induced expression of an shRNA targeting the insulin receptor (FH1tIR5UTG). Based on a stable eGFP expression tg rats were identified (Fig. 3a). Adding 2 mg/ml dox into the drinking water of tg rats, led to a increase in blood glucose levels already after four days and reached the peak at day seven (Fig. 3b). In accordance with this observation, a very high expression of the shRNA and a strong reduction of the insulin receptor protein were detected (Fig. 3c). Additionally the tetR-protein was also detected in those animals (Fig. 3d), showing that all components of this lentiviral vector are reasonably expressed and fully functional.

To further characterize the sensitivity of this model, different concentrations of dox were added to the drinking water of tg animals. Even at concentrations as low as 20 µg/ml (Fig. 4b) of dox an increase of the glucose levels in the blood of those animals was observed after seven days, which was comparable to 200 µg/ml (Fig. 4a) and 2 mg/ml of dox (Fig. 3b). Additional analysis showed that the insulin receptor protein itself was also strongly down-regulated in those experiments (Fig. 4b). In this context it is worth mentioning that tg rats just fed with water containing sucrose showed no signs of diabetes, as evidenced by (i) normal blood glucose levels, (ii) no detectable shRNA expression and (iii) a similar expression pattern of the insulin receptor. These results show that the sensitivity of this system is very high and the regulation very tight.

### Example 3: Reversible knockdown of the Insulin receptor in rats

An important feature of this lentiviral single-vector approach is the reversibility *in vivo*. The animals were therefore treated for seven days with 20 µg/ml and analyzed the blood glucose levels. Though a strong increase at day seven was observed, withdrawal of the dox out of the drinking water resulted in a reversion of the phenotype 2-3 weeks later (Fig. 5). This shows that this construct is useful for *in vivo* applications, where the reversibility of certain gene products is desired.

**Sequence Listing, Free Text**

| | |
|---|---|
| SEQ ID NO:1 | vector FH1tshRNA-UTG |
| SEQ ID NO:2 | shCD8 alpha sequence |
| SEQ ID NOs:3/4 | probes |
| SEQ ID NO:5 | sequence encoding foot-and-mouth disease virus F2A |
| | protein |
| SEQ ID NO:6 | sequence encoding equine rhinitis A virus E2A |
| | protein |
| SEQ ID NOs:7-208 | shRNA sequences |
| SEQ ID NOs:209-211 | siRNA sequences |

## Claims

1. An inducible lentiviral vector comprising
(a) a responder construct comprising at least one segment corresponding to a short hairpin RNA (shRNA) or to complementary short interfering RNA (siRNA) strands, said segment being under control of a ubiquitous promoter, wherein said promoter contains at least one operator sequence, by which said promoter is perfectly and ubiquitously regulatable by a repressor and
(b) a regulator construct comprising a codon-optimized repressor gene, which provides for perfect regulation of the promoter of the responder construct, said codon-optimized repressor gene is located upstream of a marker gene and is under the control of a ubiquitously active promoter, and said codon-optimized repressor gene and said marker gene being linked by a DNA sequence encoding a viral peptide sequence.

2. The lentiviral vector of claim 1, wherein
(i) said responder construct and said regulator construct allow inducible gene knock down in said biological entity, the regulation by said repressor permits control of the expression and the suppression of the expression of the shRNA or the siRNA by a rate of at least 90%, preferably by a rate of at least 95%, more preferably by a rate of at least 98%, and most preferably by a rate of 100%; and/or
(ii) the promoter of the responder construct is selected from RNA polymerase (Pol) I, II and III dependent promoters, preferably is a Pol II and III dependent promoter, more preferably is selected from a CMV promoter, a CAGGS promoter, a Ubiquitin promoter, a RNAse P RNA promoter such as H1, a tRNA promoter, a 7SL RNA promoter, and a 5 S rRNA promoter; and/or
(iii) the promoter of the regulator construct is selected from RNA polymerase (Pol) I, II and III dependent promoters, preferably is a Pol II and III dependent promoter, more preferably is selected from a CMV promoter, a CAGGS promoter, a Ubiquitin promoter, a snRNA promoter such as U6, a RNAse P RNA promoter such as H1, a tRNA promoter, a 7SL RNA promoter, and a 5 S rRNA promoter; and/or
(iv) the responder construct and/or the regulator construct further contain functional sequences selected from splice acceptor sequences, selectable marker sequences, recognition sequences, etc.; and/or
(v) the DNA sequence encoding a viral peptide sequence of the regulator construct is selected from viral sequences from foot-and-mouth disease virus F2A (including that of SEQ ID NO:5), equine rhinitis A virus E2A (including that of SEQ ID NO:6), porcine teschovirus-1 P2A and Thosea asigna viral T2A and most preferably is the Thosea asigna viral T2A peptide sequence having the sequence of bp 4836 to 4889 of SEQ ID NO:1; and/or
(vi) the vector is suitable to be stably integrated into the genome of a biological entity, preferably at actively transcribed regions.

3. The lentiviral vector of claim 1 or 2, wherein in the responder construct
(i) the promoter is an inducible promoter selected from polymerase (Pol) III dependent promoters, preferably is an RNAse P RNA promoter such as H1, and most preferably is a H1-promoter; and/or
(ii) the promoter contains an operator sequence selected from tetO, GalO, lacO, etc., preferably the operator sequence is tetO; and/or
(iii) the operator sequence of the promoter is positioned 1 to 10 bp, preferably1 to 2 bp 3' (i.e., downstream) and/or 5' (i.e., upstream) of the TATA element; and/or
(iv) the DNA sequence corresponding to the shRNA or siRNA is positioned 3' to said operator sequence; and/or
(v) carries a Pol III dependent promoter, preferably an inducible H1 promoter, containing the operator, such as tetO, and the segment(s) corresponding to a shRNA or siRNA; and/or
(vi) comprises at least one shRNA segment having a DNA sequence A-B-C or C-B-A, or comprises at least two siRNA segments A and C or C and A, each of said at least two siRNA segments being under the control of a separate promoter, wherein
A is a 15 to 35, preferably a 19 to 29 bp DNA sequence with at least 95%, preferably 100% complementarily to the gene to be knocked down;
B is a spacer DNA sequence having 5 to 9 bp forming the loop of the expressed RNA hair pin molecule; and
C is a 15 to 35, preferably a 19 to 29 bp DNA sequence with at least 85% complementarily to the sequence A; and/or
(vii) comprises a stop and/or a polyadenylation sequence.

4. The lentiviral vector according to any of claims 1 to 3 wherein in the regulator construct
(i) the repressor gene is under control of an ubiquitous promoter preferably selected from polymerase (Pol) I, II and III dependent promoters, preferably is a Pol II dependent promoter, more preferably is a CMV promoter, a CAGGS promoter or a ubiquitin promoter; and/or
(ii) the repressor gene is a codon-optimized tet repressor, a codon-optimized Gal4 repressor, a codon-optimized lac repressor or a variant thereof, preferably is a codon-optimized tet repressor having the sequence of bp 4206 to 4826 of SEQ ID NO:1; and/or
(iii) the marker is a fluorescence marker, preferably selected from green fluorescent protein (GFP), DsRed, AsRed, HcRed, Tomatoe, Cherry, Katushka, and variants thereof, most preferably is eGFP having the sequence shown of bp 4896 to 5609 of SEQ ID NO:1.

5. The lentiviral vector of any one of claims 1 to 4, wherein the responder construct is a H1-tetO-shRNA cassette, preferably having the sequence of bp 2594 to 2953 of SEQ ID NO:1 and/or the a regulator construct comprises a codon optimized Tet-Repressor located upstream of the eGFP marker gene and is under the control of the ubiquitin promoter, and said TetR and said marker gene being linked by the viral T2A peptide sequence, preferably said regulator construct has the sequence of bp 2974 to 5609 of SEQ ID NO:1, most preferably the lentiviral vector has the sequence shown in SEQ ID NO:1.

6. A biological entity selected from a non-human vertebrate, a tissue culture derived from a vertebrate or one or more cells of a cell culture derived from a vertebrate, said biological entity carrying a responder construct and a regulator construct as defined in claims 1 to 5, wherein the responder construct and/or the regulator construct are stably integrated into the genome of the biological entity, preferably at actively transcribed regions.

7. The biological entity according to claim 6, wherein
(i) the responder construct and/or the regulator construct is (are) stably integrated into the genome of the biological entity, preferably at actively transcribed regions, by viral integration or the like, preferably by viral transduction with a lentiviral vector of claims 1 to 5; and/or
(ii) the responder construct and the regulator construct are integrated at the same locus in the genome of the biological entity, preferably said biological entity carrying the sequence of bp 839 to 6973 of SEQ ID NO.1; and/or
(iii) the vertebrate is a non-human vertebrate, preferably is a rodent, such as mouse or rat, pig, cattle, sheep, bird or a fish, most preferably the vertebrate is a rat or mouse; and/or
(iv) the cells of the vertebrate carrying a responder construct and a regulator construct as defined in claims 1 to 5 are mammalian cells including human cells, preferably are primary cells stem cells and zygotes, but not including human stem cells and zygotes; and/or
(v) the biological entity is a rat, rat cell or ratmouse tissue, and the responder and regulator construct are as defined in claims 1 to 5.

8. A method for preparing the biological entity as defined in any of claims 6 or 7 or a method for inducible gene knock down in a biological entity, which method comprises stably integrating a responder construct and a regulator construct into the genome of the biological entity by viral transduction with a lentiviral vector as defined in claims 1 to 5.

9. The method of claim 8 which is suitable for preparing a transgenic non-human vertebrate, preferably a transgenic non-human mammal and which comprises transducing non-human ES cells with a lentiviral vector as defined in claims 1 to 5.

10. Use of a biological entity as defined in any of claims 6 or 7 for inducible gene knock down, and/or as a test system for pharmaceutical testing, and/or for gene target validation, and/or for gene function analysis.
